# EUROPEAN PATENT SPECIFICATION

(11) **EP 4 477 102 B1**
(45) Date of publication and mention of the grant of the patent: **08.07.2026**
(21) Application number: 24180997.9
(22) Date of filing: 10.06.2024
(51) Int. Cl.: A43B 17/00, A43D 1/02, A43D 1/06

(54) **INSOLE DESIGNING APPARATUS, INSOLE DESIGNING METHOD AND PROGRAM**
EINLEGESOHLENENTWURFSVORRICHTUNG, EINLEGESOHLENENTWURFSVERFAHREN UND PROGRAMM
APPAREIL DE CONCEPTION DE SEMELLE INTÉRIEURE, PROCÉDÉ ET PROGRAMME DE CONCEPTION DE SEMELLE INTÉRIEURE

(30) Priority: 14.06.2023 JP 2023097723
(43) Date of publication of application: 18.12.2024
(73) Proprietor: ASICS Corporation, Kobe-shi, Hyogo 650-8555 (JP)
(72) Inventor: HATANO, Genki, Kobe-shi 650-8555 (JP); TANIGUCHI, Norihiko, Kobe-shi 650-8555 (JP); TAKASHIMA, Shingo, Kobe-shi 650-8555 (JP)
(74) Representative: TBK

(56) References cited:
- EP-A1- 4 327 689
- WO-A1-2017/057388
- WO-A1-2020/044083
- DE-A1- 102019 108 820

## Description

### BACKGROUND

### Technical field

The present disclosure relates to an insole designing apparatus, an insole designing method, and a program.

### Background Information

Prior art which is related to this field disclosed in WO2017/057388A1 showing insole design system, shoe, and method for combining shoe and insole, in DE102019108820 A1 showing device for manufacturing an individually configured insole for a shoe, in WO2020/044083 A1 showing method for design of insoles, and in EP4327689 A1 showing sock liner designing apparatus, sock liner designing method and program.
Conventionally, insoles may be often produced to order. They are adapted to individual foot shapes and used to enhance the fit and stability of footwear like shoes and sandals. For example, WO2017/057388 describes an insole designing apparatus used to design insoles. This apparatus calculates, based on a foot shape (foot form data) and shoe data, the state of a foot placed in the inner space of a shoe and then designs an insole based on a three-dimensional void formed between the foot and the shoe's inner surface.

### SUMMARY

The insole designing apparatus described in WO2017/057388 (Patent Literature 1), however, may design and produce an insole with an intention to keep the foot in a desired state when the insole and the shoe are combined and used. Thus, this apparatus may focus on an improved fit of the shoe. However, properties of shoes desired by users may include but are not necessarily limited to such a fit. The apparatus described in WO2017/057388 (Patent Literature 1) was developed to design an insole based on the inner space of a shoe before any insole is inserted and used.

To address these issues of the known art, the present disclosure is directed to providing an insole designing apparatus, an insole designing method and a program for use in designing an insole replaceable with and used instead of an insole currently used in footwear.

The present disclosure provides an insole designing apparatus according to claim 1.

The present disclosure provides an insole designing method according to claim 12.

The present disclosure provide a program according to claim 13.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is a schematic diagram illustrating a system configuration including an insole designing apparatus according to an embodiment.
Fig. 2 is a block diagram illustrating a configuration of the insole designing apparatus according to the embodiment.
Fig. 3 is a schematic diagram for describing an example of use of an insole according to the embodiment.
Fig. 4 is a flow chart of a designing process carried out by the insole designing apparatus according to the embodiment.
Fig. 5 is a schematic diagram used for describing the contour of an insole designed by the designing process carried out by the insole designing apparatus.
Fig. 6 is a schematic diagram for describing cup heights measured in different portions of the insole according to the embodiment.
Fig. 7 is a schematic diagram for describing thicknesses of different portions of the insole according to the embodiment.
Fig. 8 is a schematic diagram for describing an arch position in the insole according to the embodiment.
Fig. 9 is a schematic diagram illustrating an insole in the form of a three-dimensional mesh structure.
Fig. 10 is a schematic diagram for describing the three-dimensional structure in a base portion of the insole of Fig. 9.
Fig. 11 is a flow chart of an adjustment process carried out by the insole designing apparatus according to the embodiment.
Fig. 12 is a schematic diagram for describing Example 1, illustrating adjustments of design data using the insole designing apparatus according to the embodiment.
Fig. 13 is a diagram for describing correlation between the insole design data and footwear properties.
Fig. 14 is a schematic diagram for describing pronation.
Fig. 15 is a schematic diagram for describing Example 2, illustrating adjustments of design data using the insole designing apparatus according to the embodiment.
Fig. 16 is a schematic diagram for describing Example 3, illustrating adjustments of design data using the insole designing apparatus according to the embodiment.
Fig. 17 is a schematic diagram for describing Example 4, illustrating adjustments of design data using the insole designing apparatus according to the embodiment.
Fig. 18 is a flow chart of a prediction process carried out to predict a subject's foot shape in an unloaded state.
Fig. 19 is a table showing an example of loaded foot shape data.
Fig. 20 is a table showing an example of unloaded foot shape data.
Fig. 21 is a diagram for describing calculation of a difference between a subject's data and first sample data.

### DETAILED DESCRIPTION

Embodiments are hereinafter described with reference to the accompanying drawings. In the description below, the same configurations are illustrated with the same reference signs. Also, they are referred to likewise and have similar functional features. Such configurations, therefore, will not be repeatedly described in detail. In the description of embodiments given below, the "medial side" or "medial foot side" refers to a side of foot closer to the "first toe" in the direction of foot width, while the "lateral side" or "lateral foot side" refers to a side of foot closer to the "fifth toe" in the direction of foot width.

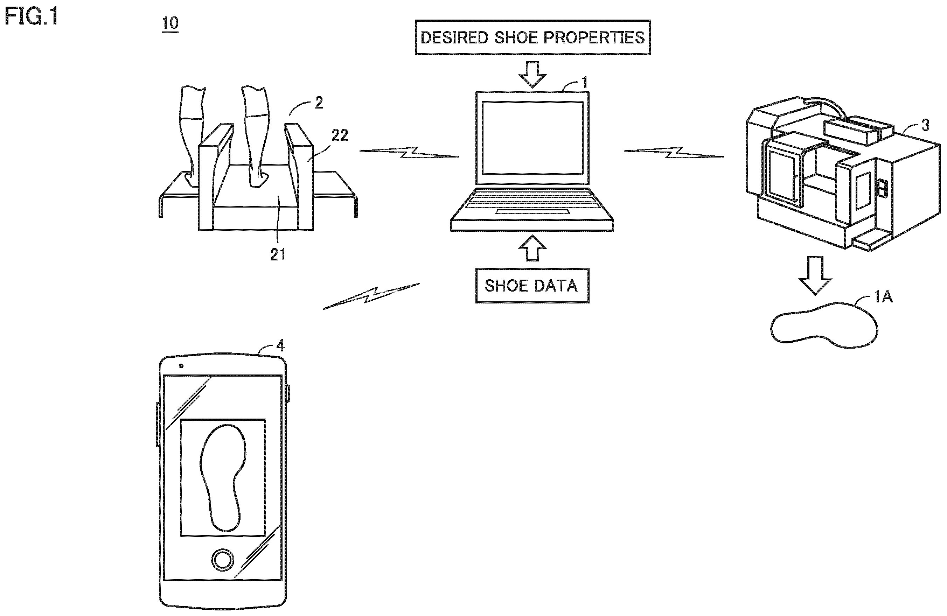

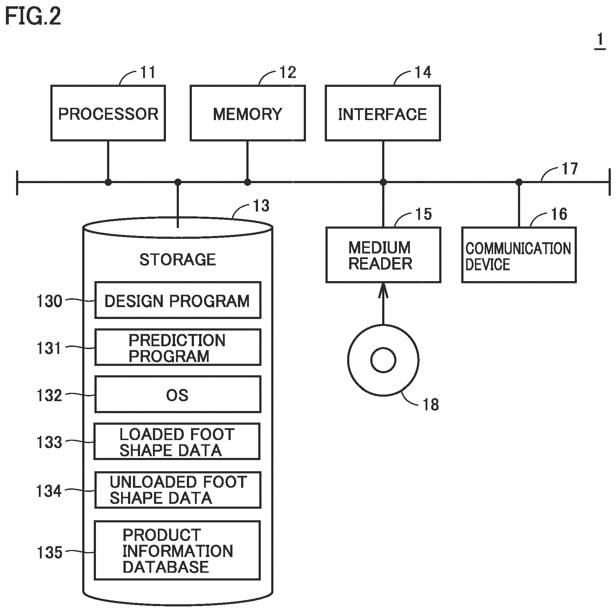

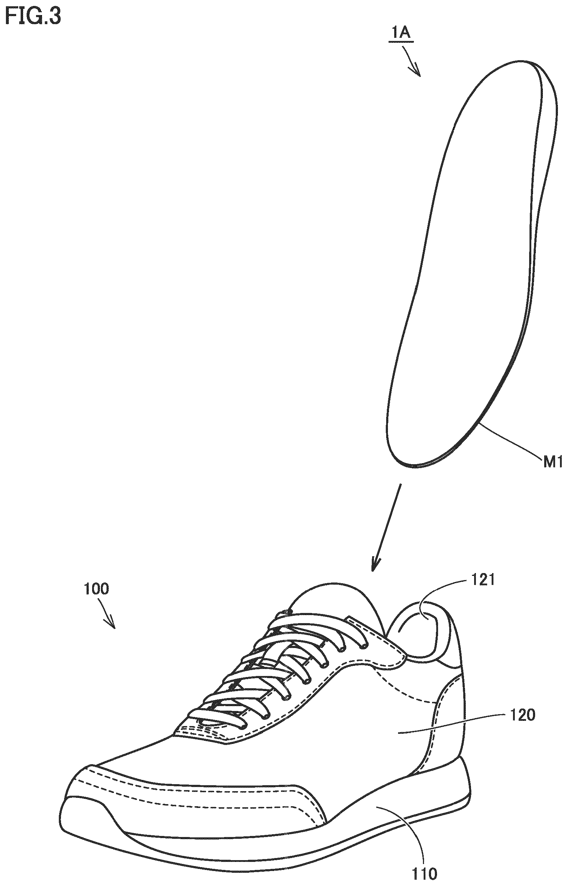

Fig. 1 is a schematic diagram illustrating a system configuration including an insole designing apparatus 1 according to an embodiment. Fig. 2 is a block diagram illustrating a configuration of the insole designing apparatus 1 according to the embodiment. Fig. 3 is a schematic diagram for describing an example of use of an insole 1A according to the embodiment. In shops like footwear stores or event venues, custom-made insoles, like an insole 1A, may be produced to order. The insole 1A is inserted and used in footwear 100, an example of which is a shoe, as illustrated in Fig. 3. The footwear 100 has a sole 110 and an upper 120. In use, the insole 1A is inserted into the footwear 100 through a top line 121 formed on the upper 120. The insole 1A is inserted in the footwear 100 and placed on its inner bottom surface, with the insole's lower surface facing the footwear's inner bottom surface. The insole 1A is thus attached to the footwear 100. In the description given below, examples of the footwear 100 to which the insole 1A is attached may include shoes and sandals.

While a user is wearing the footwear 100, the sole of the user's foot is resting on the upper surface of the insole 1A. The insole 1A is pressed by the sole of the user's foot and the sole 110 of the footwear 100 and, is thus allowed to support the user's foot. While the user's foot is staying in the inner space of the footwear 100, the insole 1A fills a void formed between the foot and the inner space of the footwear 100, allowing a better fit of the footwear 100.

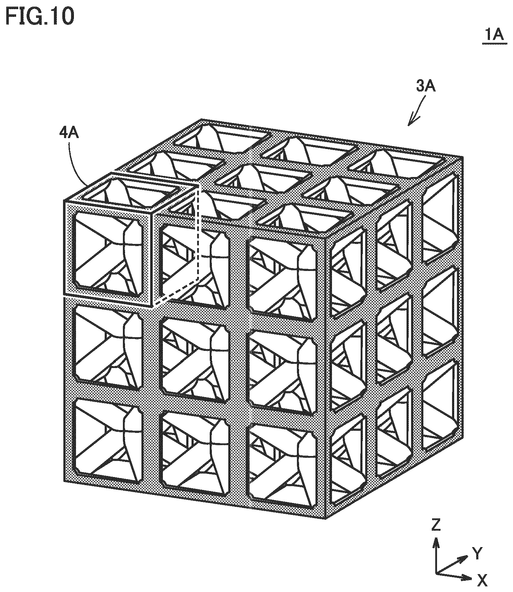

To improve the footwear's fit, the inside of the footwear 100 and a contour M1 of the insole 1A preferably conform in size and shape to each other. This may avoid any displacement of the insole (1A) in the footwear 100. The inside of the footwear 100 and the contour M1 of the insole 1A may be thus allowed to conform in size and shape to each other by preparing a different one of the insoles 1A for each size of the footwear 100. However, such an exact match of the contour M1 of the insole 1A to the inside of the footwear 100 may be often difficult because the footwear 100 may variously differ in size and shape. Expertise skills may be required to manually cut out the insole 1A and have its contour M1 to match the inside of the footwear 100. The insole 1A may have, for example, a three-dimensional mesh structure as described later. When the insole 1A thus structured is cut along its contour, a unit structure(s) along the cut end (see Fig. 10) may be partly broken. To avoid that, the cut ends should be subjected to a certain process(s). In the insole designing apparatus 1 according to this embodiment, therefore, design data of the insole 1A including the contour M1 in contact with the inside of the footwear 100 is calculated based on foot form data (first data) associated with a user's foot shape and inside data (second data) associated with the inside of the footwear 100.

The insole designing apparatus 1 designs the insole 1A that offers a better fit of the footwear 100 and further adjusts the design data of the insole 1A (for example, material, structure and cup height and thickness in each portion) to change properties of the footwear 100 (for example, cushioning performance, stability, grip). The insole designing apparatus 1 thus characterized may successfully design the insole 1A that can achieve properties (footwear properties) required of the footwear 100 by a user. The design data of the insole 1A may be adjusted in order to change properties of the footwear 100. This, however, is not an indispensable matter required of the insole designing apparatus, insofar as this apparatus is at least configured to calculate the design data of the insole 1A including the contour M1 in contact with the inside of the footwear 100.

### [Configuration of insole producing system]

An insole producing system 10 is hereinafter described. This system is used to produce the insole 1A designed by the insole designing apparatus 1. As illustrated in Fig. 1, the insole producing system 10 includes the insole designing apparatus 1, a measuring apparatus 2, a 3D printer 3, and an information terminal 4. The apparatus used to produce the insole 1A is not necessarily limited to the 3D printer 3 and may be selected from other apparatuses, an example of which is an NC working machine. This embodiment describes the use of a dedicated designing apparatus, the insole designing apparatus 1, to prepare the design data of the insole 1A. Optionally, the technology disclosed herein may be applied to an available designing apparatus for custom-made footwear production and have this apparatus prepare the design data of the insole 1A.

The measuring apparatus 2 may be, for example, a 3D foot form scanner using laser. This apparatus includes a top board 21 and laser measuring units 22 disposed on sides across the top board. When a subject (user) in the upright position rests subject's foot on the top board 21, a load is applied from the foot onto the top board 21 under the subject's weight. This may be rephrased that the load is being imposed on the subject's foot. The measuring apparatus 2, with the load being thus imposed on the subject's foot, measures subject's foot shape using the laser measuring unit 22 while moving from the toe to heel. The measuring apparatus 2 outputs, to the insole designing apparatus 1, the subject's data including subject's foot shape data (foot form data) obtained by the laser measuring units 22. The subject's data only needs to include at least the foot shape data obtained by the measuring apparatus 2 and may further include other pieces of data (for example, personal data such as the subject's gender and/or age).

In addition to the foot shape data of a target subject for measurement, the inside data associated with the inside of the footwear 100 is obtained using the information terminal 4. The information terminal 4 has a functional feature for generating the shape of a measurement target using, for example, LiDAR (Light Detection And Ranging)-leveraged three-dimensional scan or image synthesis like photogrammetry. To this end, the information terminal 4 obtains data of a scanned or photographed shape of the insole of the footwear 100 (original insole of the footwear 100 when purchased), as illustrated in Fig. 1. The data of the scanned or photographed insole's shape contains data of a portion of the insole in contact with the inside of the footwear 100, i.e., data of the contour shape of the insole in contact with the inside of the footwear 100.

The contour shape data of the insole in contact with the inside of the footwear 100 may be easily obtained when the insole is removed from the footwear 100 to scan or photograph the insole shape using the information terminal 4. The contour shape data of the insole in contact with the inside of the footwear 100 may be obtained otherwise, for example, by scanning or photographing the inside of the insole-used footwear 100 using the information terminal 4. To scan or photograph the insole shape, the insole may be placed on a mat or a piece of paper of a known size, or an augmented reality tool may be used to achieve a higher shape accuracy. The information terminal 4 may be handled by a user or by a store staff. The information terminal 4 may be a terminal owned by a store (for example, smartphone or tablet terminal) or may be a terminal owned by a user (for example, smartphone or tablet terminal). Any distortion of the data obtained by scanning or photographing may be corrected using camera parameters obtained from hardware information of the information terminal 4. The apparatus used to obtain the contour shape data of the insole in contact with the inside of the footwear 100 is not necessarily limited to the information terminal 4 and may be selected from other apparatuses. Examples of such apparatuses may include X-ray CT apparatuses and three-dimensional measuring apparatuses. The former apparatus captures inner cross-sectional images of the footwear 100, while the latter apparatus measures mold obtained from, for example, a resin material poured into and cured in the footwear 100.

The insole designing apparatus 1 receives the foot shape data (foot form data) from the measuring apparatus 2 and further receives the inside data associated with the inside of the footwear 100 from the information terminal 4, and then calculates the design data of the insole 1A based on the foot shape data and the inside data. The insole designing apparatus 1 is allowed to adjust the design data to design the insole 1A that can fulfill properties of the footwear 100 required by a user, which will be described later. The insole designing apparatus 1 outputs the generated design data of the insole 1A to, for example, the 3D printer 3. The 3D printer 3 produces the insole 1A based on the design data of the insole 1A.

Thus, the insole producing system 10 calculates, using the insole designing apparatus 1, design data of the insole 1A including the contour of the insole 1A in contact with the inside of the footwear 100 based on the foot shape data (foot form data) of a subject for measurement (user) obtained by the measuring apparatus 2 and the inside data obtained by the information terminal 4. Then, this system produces the insole 1A based on the calculated design data using the 3D printer 3. Thus, the insole producing system 10 may successfully design the insole 1A using the insole designing apparatus 1 so that the inside of the footwear 100 and the contour M1 of the insole 1A conform in size and shape to each other and then easily produce the insole 1A using the 3D printer 3.

In the present disclosure, the insole 1A is produced by the insole producing system 10 having the insole designing apparatus 1, the measuring apparatus 2, the 3D printer 3, and the information terminal 4. Such structural components may be a few examples that constitute the insole producing system. The insole producing system 10 may be structured so that the insole designing apparatus 1, the measuring apparatus 2 and the 3D printer 3 are integrally combined, or may be structured so that the measuring apparatus 2 and the 3D printer 3 are integrally combined. In the present disclosure, instead of the whole insole 1A being produced by the 3D printer 3 as described earlier, the insole 1A may be only partly produced by the 3D printer 3. To be specific, premade parts and other parts made by the 3D printer 3 may be optionally combined into a custom-made insole. Optionally, such premade parts may be selectively used based on information of, for example, individual foot shapes.

### [Configuration of insole designing apparatus]

The structural features of the insole designing apparatus 1 are described in further detail. As illustrated in Fig. 2, the insole designing apparatus 1 has a processor 11, a memory 12, a storage 13, an interface 14, a medium reader 15, and a communication device 16. These devices and units are interconnected through a processor bus 17.

The processor 11 is an example of the "processing circuitry". The processor 11 is a computer that reads out a program stored in the storage 13 (for example, design program 130, prediction program 131 and OS (Operating System) 132) and then loads and runs the read program in the memory 12. The processor 11 may include, for example, CPU (central processing unit), FPGA (field programmable gate array), GPU (graphics processing unit) or MPU (Multi Processing Unit). The processor 11 may include a processing circuitry.

The memory 12 includes a volatile memory, for example, DRAM (dynamic random access memory) or SRAM (static random access memory), or may include a non-volatile memory, for example, ROM (read only memory) or flash memory.

The storage 13 is an example of the "storage". The storage 13 may include a non-volatile storage device, for example, HDD (Hard Disk Drive) or SSD (Solid State Drive). In the storage 13 are stored programs including, for example, a design program 130, loaded foot shape data 133, unloaded foot shape data 134, and a product information database 135.

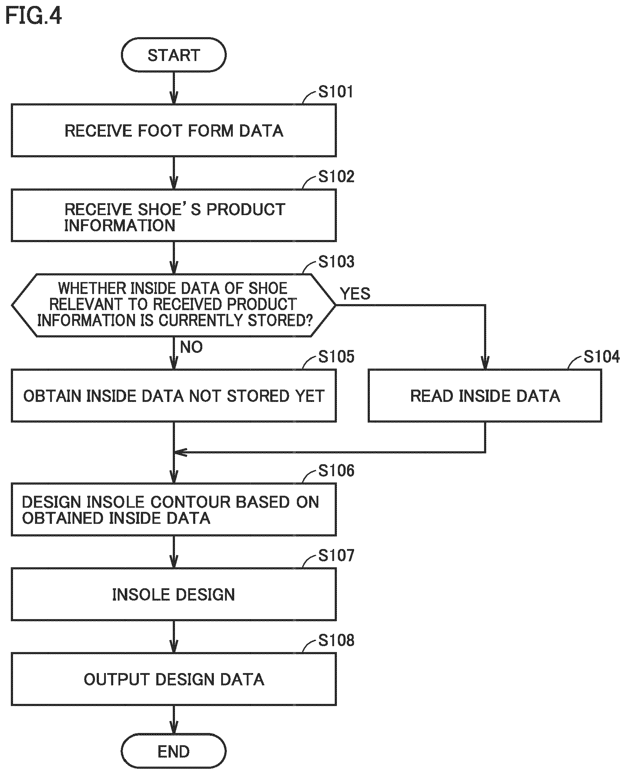

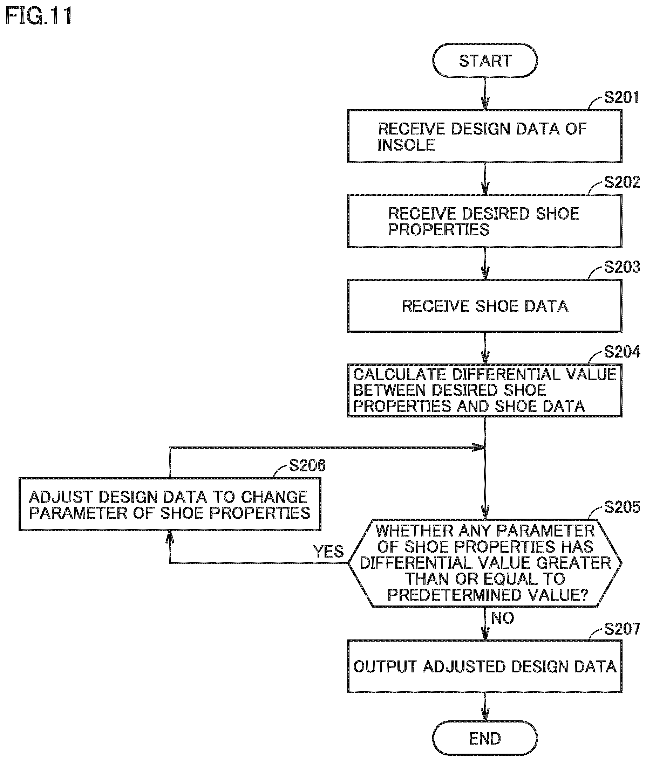

The design program 130 is a program run to execute a process to obtain design data of the insole 1A based on the foot shape data and inside data (designing process of Fig. 4 described later) and a process to adjust the design data in order to produce the insole 1A that can fulfill properties required of the footwear 100 by a user (adjustment process of Fig. 11 described later).

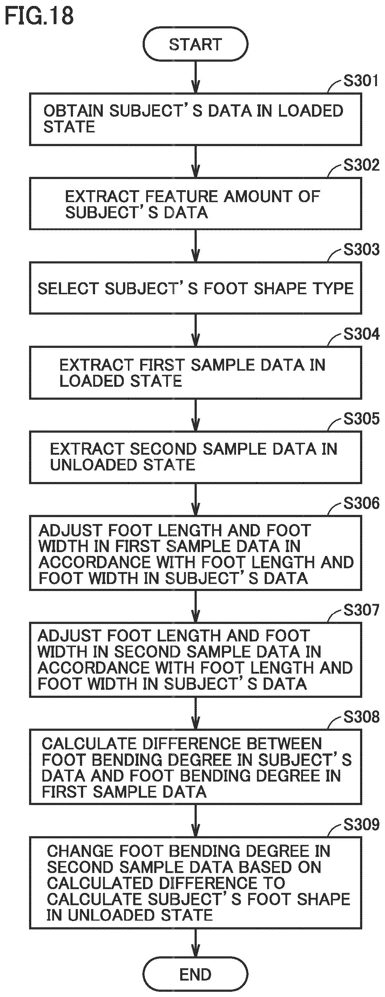

The prediction program 131 is a program run to execute a process to predict a user's foot shape in the unloaded state or low-load state (hereinafter, may be simply referred to as unloaded state). The prediction program 131 is a program run to execute a process, using the insole designing apparatus 1, to predict a user's foot shape in the unloaded state (prediction process of Fig. 18 described later) based on the user's foot shape in a loaded state obtained by the measuring apparatus 2.

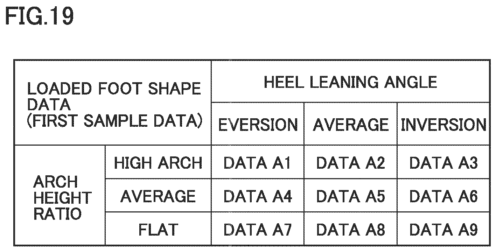

The loaded foot shape data 133 is data used to predict a user's foot shape in the unloaded state. The loaded foot shape data 133 is an example of the "first sample data", which contains data calculated from pieces of foot shape data of a plurality of samples in the loaded state. The loaded foot shape data 133 will be described later in detail with reference to Fig. 19.

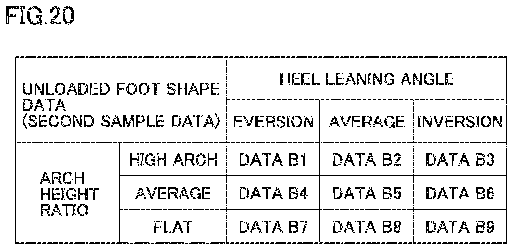

The unloaded foot shape data 134 is used to predict a user's foot shape in the unloaded state. The unloaded foot shape data 134 is an example of the "second sample data", which contains data calculated from pieces of foot shape data of a plurality of samples in the unloaded state. The unloaded foot shape data 134 will be described later in detail using Fig. 20.

The product information database 135 is a database in which the inside data of the footwear 100 is storable in association with product information of the footwear 100 (for example, product name, model number). The product information database 135 is not necessarily stored in the storage 13. This database may instead be stored on a cloud server or in another computer connected to a network through the communication device 16.

The interface 14 is an example of the "input device" or "output device". The interface 14 is used to receive data inputs from an operator of the insole designing apparatus 1 through a keyboard, mouse and/or touch device and is also used to output, to a display device, the design data designed by the insole designing apparatus 1.

The medium reader 15 is configured to receive a recording medium, for example, a removable disc 18 and then obtain data stored in this removable disc 18.

The communication device 16 is an example of the "input device" or "output device". The communication device 16 transmits and receives data to and from other devices through wireless or wired communication. The communication device 16 communicates with the measuring apparatus 2 and the information terminal 4 to receive the foot shape data from the measuring apparatus 2 and also receive the inside data from the information terminal 4. The communication device 16 communicates with the 3D printer 3 to output, to the 3D printer 3, the design data of the insole 1A used to produce the insole 1A.

The insole designing apparatus 1 does not necessarily receive data including the foot shape data and inside data through the communication device 16. For example, the insole designing apparatus 1 may receive the data including the foot shape data and inside data through the interface 14. Otherwise, the insole designing apparatus 1 may read data including the foot shape data and inside data stored in the removable disc 18 using the medium reader 15.

The removable disc 18, examples of which may include CD-ROM and DVD (Digital Versatile Disc), is not the only option as a medium in which programs run by the processor 11 of the insole designing apparatus 1 are storable. The medium may be optionally selected from semiconductor memories, for example, IC card, optical card, mask ROM, EPROM (Erasable Programmable ROM) and EEPROM (Electrically EPROM).

Programs to be run by the processor 11 of the insole designing apparatus 1 may include but are not necessarily limited to programs stored in the removable disc 18. Instead, a program(s) stored in the storage 13 may be downloaded into and run in the RAM. In this instance, another computer connected to a network through the communication device 16 may rewrite a program(s) stored in the storage 13 or may write a new program(s) and adds it to the storage 13. The insole designing apparatus 1 may download a program(s) from another computer connected to a network through the communication device 16 and store the downloaded program(s) in the storage 13. The program described herein may include a program(s) that can be directly run by the processor 11 of the insole designing apparatus 1 and also include other programs, for example, a source program(s), a compressed program(s), and an encrypted program(s).

### [Generation of design data of insole]

The designing process to generate the design data of the insole 1A using the insole designing apparatus 1 is hereinafter described with reference to Fig. 4. Fig. 4 is a flow chart of the designing process carried out by the insole designing apparatus 1 according to the embodiment. Steps illustrated in Fig. 4 (hereinafter, step numbers starting with "S") are carried out by having the design program 130 run by the processor 11 of the insole designing apparatus 1.

As illustrated in Fig. 4, the insole designing apparatus 1 receives the foot form data obtained by the measuring apparatus 2 (S101). The insole designing apparatus 1 receives product information of the footwear 100 in which the insole 1A will be used (product name, model number (S102). The insole designing apparatus 1 has the product information database 135 in which the inside data of the footwear 100 is storable in association with the product information of the footwear 100 (for example, product name, model number). The insole designing apparatus 1 receives, in S102, input of the product information of the footwear 100 to check whether the received product information is relevant to the footwear 100 whose inside data is currently stored in the product information database 135.

The insole designing apparatus 1 checks, in regard to the received product information of the footwear 100, whether the product information database 135 is containing the inside data of this footwear 100 (S103). When the earlier step determines that the inside data is currently stored in the product information database 135 (YES in S103), the insole designing apparatus 1 reads out the inside data relevant to the footwear 100 of the received product information from the product information database 135 (S104). When the earlier step determines that the inside data is currently not stored in the product information database 135 (NO in S103), the insole designing apparatus 1 newly obtains inside data of an insole shape in the footwear 100 captured by scanning or photographing using the information terminal 4 (S105). The insole designing apparatus 1 stores the newly obtained inside data in the product information database 135 in association with the product information of the footwear 100. Thus, any inside data, if the relevant footwear 100 is the same product as the footwear 100 whose inside data has been obtained and stored, is no longer necessary.

In case the footwear 100 relevant to the received product information differ in size to the footwear 100 of the inside data stored in the product information database 135, the insole designing apparatus 1 may, for example, perform similitude transformation of the inside data to adjust the size of the footwear 100 in this data. The data conversion applicable to the inside data for different sizes is not necessarily limited to the similitude transformation and may be selected from the known techniques, for example, applying scale ratio to each piece of data. Thus, the product information database 135 may only be required to store the inside data of one size for the same footwear 100, instead of the inside data of all sizes.

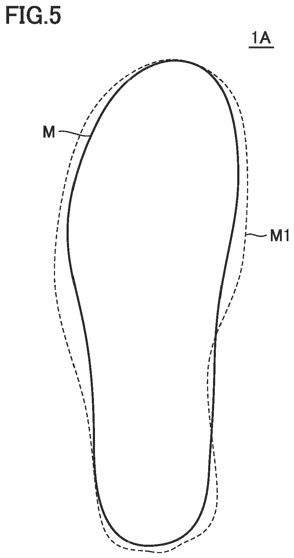

The insole designing apparatus 1 calculates design data of the contour M1 of the insole 1A based on the inside data of the footwear 100 (S106). Specifically, the insole designing apparatus 1 designs the contour M1 of the insole 1A so that the inside of the footwear 100 and the contour M1 of the insole 1A are equal in size and shape to each other. Fig. 5 is a schematic diagram used for describing the contour M1 of the insole designed by the designing process carried out by the insole designing apparatus 1 according to the embodiment. As illustrated in Fig. 5, the insole designing apparatus 1 selects a standard contour M of the insole 1A depending on the size of the footwear 100 and then reforms the contour M to exactly follow the inside of the footwear 100 based on the inside data of the footwear 100, to design the contour M1. Whether the contour M is equal in size and shape to the inside of the footwear 100 may be evaluated by visualizing an average distance from the contour M to the inside of the footwear 100.

Then, the insole designing apparatus 1 generates the design data of the insole 1A based on the foot form data received in S101 (S107). The design data of the insole 1A generated in this step is design data in which the shape of the insole 1A is optimized in accordance with a shape indicated by the foot form data. This design data differs from the design data of the insole 1A adjusted to the footwear properties as described later. The process of S107, using a known means or technique, generates the design data of the insole 1A adjusted to fit the foot shape.

When the adjustment process described later is not performed, the insole designing apparatus 1 outputs the generated design data of the insole 1A to the 3D printer 3 (or NC working machine) (S108). The 3D printer 3 (or NC working machine) makes the insole 1A based on the generated design data of the insole 1A.

### [Adjustment parameter]

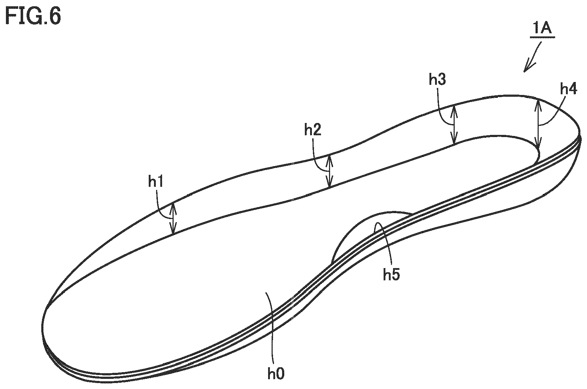

The insole 1A can be optimized in shape, specifically, in length and width, if the insole 1A should only be designed to serve a better fit of the footwear 100. To allow the insole 1A in the footwear 100 to desirably change the properties of the footwear 100 (for example, cushioning performance, stability, grip), however, the material, structure, cup height and thickness in each portion of the insole 1A may need to be optimally adjusted. The parameters included in the design data of the insole 1A may be roughly divided into parameters for shape and parameters for adjustment. The parameters for shape refer to shape-related parameters including the length and width of the insole 1A. The parameters for adjustment refer to parameters that may affect the footwear properties including the material, structure, cup height and thickness in each portion. The parameters for adjustment are hereinafter described with reference to Figs. 6 to 10. The properties of the footwear 100 may be changeable by adjusting, in the design data of the insole 1A, at least one of the following parameters as the parameter for adjustment; material, structure, cup height in each portion, insole scale ratio, thickness in each portion, arch position and bottom surface shape.

Of the adjustment parameters includable in the design data of the insole 1A, the material refers to a material(s) used to make the insole 1A. Through adjustment of the material used, some or all of the required properties including: cushioning performance, grip, bendability, durability, repulsion, and lightness in weight, may be conferred on an insole to be produced. When the insole 1A is made of a resin material(s), one or more of the following examples may be selected; polyolefin resins, ethylene-vinyl acetate copolymers (EVA), polyamide-based thermoplastic elastomers (TPA, TPAE), thermoplastic polyurethanes (TPU), and polyester-based thermoplastic elastomers (TPEE). In case a rubber is the material of the insole 1A, butadiene rubber (BR), for example, may be selected and used. In case any polymer addable to polymer compositions is used as the material of the insole 1A, for example, an olefin-based polymer may be selected from olefin-based elastomers and olefin-based resins. The olefin-based polymer may be selected from polyolefines, specific examples of which may include polyethylenes (e.g., linear low-density polyethylene (LLDPE)), high-density polyethylenes (HDPE), polypropylenes, ethylene-propylene copolymers, propylene-1-hexane copolymers, propylene-4-methyl-1-pentene copolymers, propylene-1-butene copolymers, ethylene-1-hexane copolymers, ethylene-4-methyl-pentene copolymers, ethylene-1-butene copolymers, 1-butane-1-hexane copolymers, 1-butene-4-methyl-pentene, ethylene-methacrylate copolymers, ethylene-methacrylate methyl copolymers, ethylene-methacrylate ethyl copolymers, ethylene-methacrylate butyl copolymers, ethylene-methyl acrylate copolymers, ethylene-ethyl acrylate copolymers, ethylene-butyl acrylate copolymers, propylene-methacrylate copolymers, propylene-methacrylate methyl copolymers, propylene-methacrylate ethyl copolymers, propylene-methacrylate butyl copolymers, propylene methyl acrylate copolymers, propylene ethyl acrylate copolymers, propylene-butyl acrylate copolymers, ethylene-vinyl acetate copolymers, and propylene-vinyl acetate copolymers.

The polymer mentioned above may be an amide-based polymer selected from amide-based elastomers and amide-based resins. Specific examples of the amide-based polymer may include polyamide 6, polyamide 11, polyamide 12, polyamide 66, and polyamide 610.

The polymer may be selected from ester-based polymers, for example, ester-based elastomers and ester-based resins. Specific examples of the ester-based polymers may include polyethylene terephthalate and polybutylene terephthalate.

The polymer may be selected from urethane-based polymers, for example, urethane-based elastomers and urethane-based resins. Specific examples of the urethane-based polymers may include polyester-based polyurethanes and polyether-based polyurethanes.

The polymer may be selected from styrene-based polymers, for example, styrene-based elastomers and styrene-based resins. Specific examples of the styrene-based elastomer may include styrene-butylene copolymers (SEB), styrene-butadiene-styrene copolymers (SBS), hydrogenated SBS (styrene-ethylene-butylene-styrene copolymers (SEBS), styrene-isoprene-styrene copolymers (SIS), hydrogenated SIS (styrene-ethylene-propylene-styrene copolymers (SEPS), styrene-isobutylene-styrene copolymers (SIBS), styrene-butadiene-styrene butadiene (SBSB), and styrene-butadiene-styrene butadiene-styrene (SBSBS). Specific examples of the styrene-based resins may include acrylonitrile styrene resins (AS) and acrylonitrile butadiene styrene resins (ABS).

Specific examples of the polymer may include: acrylic polymers such as polymethacrylate methyl, urethane-based acrylic polymers, polyester-based acrylic polymers, polyether-based acrylic polymers, polycarbonate-based acrylic polymers, epoxy-based acrylic polymers, conjugated diene polymer-based acrylic polymers and hydrogenated materials thereof; urethane-based methacrylic polymers, polyester-based methacrylic polymers, polyether-based methacrylic polymers, polycarbonate-based methacrylic polymers, polyester-based urethane acrylates, polycarbonate-based urethane acrylates, polyether-based urethane acrylates, epoxy-based methacrylic polymers, conjugated diene polymer-based methacrylic polymers and hydrogenated materials thereof; polyvinyl chloride-based resins; silicone-based elastomers; butadiene rubbers, isoprene rubbers (IR); chloroprene rubbers (CR); natural rubbers (NR); styrene butadiene rubbers (SBR); acrylonitril butadiene rubbers (NBR); and butyl rubbers (IIR).

The material of the insole 1A may be selected from biodegradable materials or composite materials (for example, materials in which carbon fiber, glass fiber, rubber and/or resin are combined). As for the material used to produce the insole 1A, the whole insole 1A may be made of one material, the insole 1A may be made of one material, with the degree of hardness being changed in the insole's different portions, or different portions of the insole 1A may be made of materials that differ in properties.

Of the adjustment parameters includable in the design data of the insole 1A, the cup height in each portion refers to a side wall height in each portion of the insole 1A measured from the inner bottom surface of this insole. Adjustment of the cup height in each portion may confer required properties of the insole 1A including: fit, stability, support against rapid moves and turns, and arch support on this insole. Fig. 6 is a schematic diagram for describing cup heights measured in different portions of the insole 1A according to the embodiment. As illustrated in Fig. 6, the insole 1A has the following heights in different positions: a side wall height h1 measured from an inner bottom surface h0 of the insole 1A in a lateral forefoot portion, a side wall height h2 measured from the inner bottom surface h0 of the insole 1A in a lateral midfoot portion, and a side wall height h3 measured from the inner bottom surface h0 of the insole 1A in a lateral rearfoot portion. The insole 1A further has the following heights: a side wall height h4 measured from the inner bottom surface h0 of the insole 1A at a rear heel position, and a side wall height h5 measured from the inner bottom surface h0 of the insole 1A at a medial arch position.

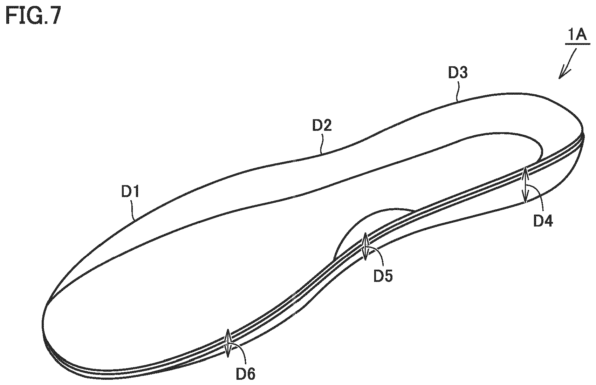

Of the adjustment parameters includable in the design data of the insole 1A, the thickness in each portion refers to the thickness of the insole 1A measured in each portion of this insole. Through adjustment of the thickness in each portion, some or all of the required properties including: cushioning performance, bendability, fit, lightness in weight, repulsion, and support against rapid moves and turns, are conferred on an insole to be produced. Fig. 7 is a schematic diagram for describing thicknesses of different portions of the insole 1A according to the embodiment. As illustrated in Fig. 7, the insole 1A has a thickness D1 at a lateral forefoot portion of the insole 1A, thickness D2 at a lateral midfoot portion of the insole 1A, and a thickness D3 at a lateral rearfoot portion of the insole 1A. The insole 1A further has a thickness D4 at a medial rearfoot portion of the insole 1A, a thickness D5 at a medial midfoot portion of the insole 1A, and a thickness D6 at a medial forefoot portion of the insole 1A.

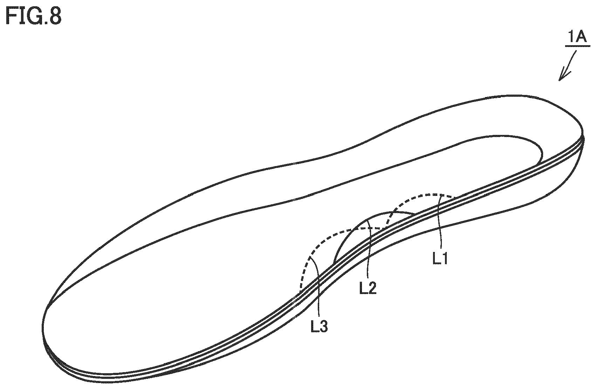

Of the adjustment parameters includable in the design data of the insole 1A, the arch position refers to the arch position in the insole 1A at which the arch of foot is supportable. Fig. 8 is a schematic diagram for describing arch positions in the insole 1A according to the embodiment. As illustrated in Fig. 8, the insole 1A has the following arch positions: L1 at which the arch is positioned on the rearfoot side, L2 at which the arch is positioned on the midfoot side, and L3 at which the arch is positioned on the forefoot side. The three arch positions L1 to L3 are simply illustrated in Fig. 8, so that the arch positions can be clearly and easily grasped. Normally, an arch top position is decided based on a foot shape obtained by predictive transform of a user's foot shape, and the arch position is then calculated and identified.

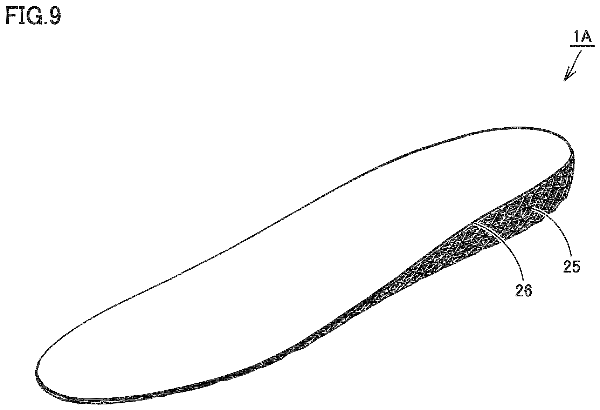

Of the adjustment parameters included in the design data of the insole 1A, the structure indicates whether the insole 1A has a flat shape or a planar structure in the form of gyroid or lattice (three-dimensional mesh structure). The structural adjustments may confer some or all of the required properties including: cushioning performance, stability, grip, bendability, durability, breathability, lightness in weight, repulsion, and support against rapid moves and turns, on an insole to be produced. Fig. 9 is a schematic diagram illustrating the insole 1A in the form of a three-dimensional mesh structure. With reference to Fig. 9, the insole 1A in the form of a three-dimensional mesh structure has a bilayer structure including a base portion 25 and an upper portion 26. The three-dimensional mesh structure of the insole 1A may be formed using a three-dimensional additive manufacturing method, for example, stereolithography.

The base portion 25 has a three-dimensional mesh structure in which a plurality of unit structures; each being a 3D lattice structure, are arranged repeatedly. Fig. 10 is a schematic diagram for describing the three-dimensional structure in the insole's base portion of Fig. 9. As illustrated in Fig. 10, the base portion 25 of the insole 1A has a three-dimensional mesh structure 3A in which a plurality of unit structures 4A; each being a 3D lattice structure, are arranged repeatedly. To be more specific, a plurality of unit structures 4A are arranged repeatedly and continuously in a regular pattern along a direction of width (X direction in the drawing), a direction of depth (Y direction in the drawing) and a direction of height (Z direction in the drawing). Fig. 10 presents only three unit structures 4A adjacent to one another along the directions of width, depth and height, and its fracture cross-sectional surfaces are illustrated in a dark color.

The lattice-like unit structure 4A has a three-dimensional shape in which a plurality of pillars extending in predetermined directions are connected to one another. The unit structure 4A may be employed from various examples including cuboidal lattice, diamond lattice, octahedron lattice, double-pyramid lattice, and these lattice structures provided with variously different supports. The unit structure 4A illustrated in the drawing is a center support-added cuboidal lattice.

The insole 1A with such a base portion 25 on its lower side may be flexibly deformable. The insole 1A may thus excel in shock absorbency, comfortableness, and stability for a user who is wearing this insole. The base portion 25 thus structured may lead to weight reduction of the insole in proportion to its size and may allow the insole to improve in breathability.

One of the adjustment parameters includable in the design data of the insole 1A is the insole scale ratio. Through adjustments of the insole scale ratio, the required properties including stability, fit, and arch support, may be imparted to the insole depending on properties required of the insole. The insole scale ratio refers to the scale ratio of a space formed by the foot shape and the inner bottom surface of the insole 1A, and 100% indicates the whole space being completely occupied and 0% indicates a state in which a portion of the insole corresponding to the arch of the foot is flat. In other words, 0% indicates a state in which the insole does not protrudes toward the arch of the foot. The adjustment parameters includable in the design data of the insole 1A disclosed herein are just exemplified ones and may include other parameters.

The parameters for shape included in the design data of the insole 1A may include the insole's bottom surface shape. The bottom surface shape may represent, for example, irregularities on the outer bottom surface of the insole 1A. When the footwear 100 to wear the insole 1A is decided, the bottom surface shape of the insole 1A is decidable correspondingly to the inner bottom surface of the footwear 100. The bottom surface of the insole 1A may have, in its end portion, a curving shape in view of production efficiency and user-friendliness.

### [Adjustment of insole design data]

The adjustment process to adjust the design data of the insole 1A using the insole designing apparatus 1 is hereinafter described, with reference to Figs. 11 to 17. Fig. 11 is a flow chart of the adjustment process carried out by the insole designing apparatus 1 according to the embodiment. Steps illustrated in Fig. 11 (hereinafter, step numbers starting with "S") are carried out by having the design program 130 run by the processor 11 of the insole designing apparatus 1.

As illustrated in Fig. 11, the insole designing apparatus 1 receives the design data of the insole 1A generated by the designing process of Fig. 4 (S201). The design data of the insole 1A generated here has been optimized so that the length and width of the insole 1A is optimized based on the foot form data and so that the contour M1 of the insole 1A conforms in size and shape to the inside of the footwear 100 based on the inside data. Specifically, the design data of the insole 1A is obtained by modelling as follows. First, a base model of the insole 1A most approximate to the inner curving shape of the footwear 100 is selected based on the obtained inside data, and this base model is enlarged/reduced based on the foot form data. Then, the curving shape of the contour M is divided into a plurality of points of control, and the base model is deformed so that the interval from each point of control to the inside of the footwear 100 is reduced to a shortest distance. The contour M1 of the insole 1A and the inside of the footwear 100 may be an exact match to each other or may be a substantial match with a small marginal space therebetween.

Further, the insole 1A is so designed that properties of the footwear 100 are changeable when this insole is worn. To this end, it is necessary to adjust the parameters for adjustment included in the design data of the insole 1A received earlier. In the description given below, the insole 1A is designed regardless of the upper material and modulus of elasticity. Such parameters associated with the inner space of the footwear 100; the upper material and modulus of elasticity, however, may naturally be considered at the time of designing the insole 1A. The insole designing apparatus 1 receives the footwear properties desired by a user (first property parameter) (S202). The footwear properties may include at least one of the following property parameters; cushioning performance, stability, grip, bendability, fit, durability, breathability, lightness in weight, repulsion, support against rapid moves and turns, and arch support. The property parameters of the footwear properties disclosed herein are just a few examples. Other property parameters may instead be used or may be used in addition to some or all of the parameters described herein.

The property parameters includable in the footwear properties are hereinafter described in detail. The property parameters includable in the properties of footwear may already be set beforehand by the time when the designing and development of the footwear are finished. Of the property parameters includable in the footwear properties, the cushioning performance indicates the shock absorbency of the footwear 100 when the footwear 100 hits the ground. The cushioning performance may be evaluated based on a performance evaluation result in terms of design and may also be evaluated in five grades of any impact based on the sensation of a user when the user's foot wearing the footwear 100 hits the ground. The cushioning performance may be evaluated using a sensor attached to a user's body or the footwear 100 or may be evaluated by measuring, using a measuring device, a force acting upon the sensor when a user's foot wearing the footwear 100 hits the ground.

Of the property parameters includable in the footwear properties, the stability indicates levels of performance of the footwear 100, for example, any horizontal displacement relative to the ground surface when the footwear 100 landed thereupon or any sideways lean on the frontal plane toward a lateral side(s) relative to the ground surface. The stability may be evaluated based on a performance evaluation result in terms of design and may also be evaluated in five grades of any lean on the frontal plane or in five grades of any directional displacement relative to the horizontal direction based on the sensation of a user's foot wearing the footwear 100 when landing on the ground. The stability may be evaluated by measuring, using image analysis or a sensor attached to a user's body or the footwear 100, any directional displacement relative to the horizontal direction based on the sensation of a user's foot wearing the footwear 100 when landing on the ground or may be evaluated by measuring any sideways lean on the frontal plane based on displacements, angles and/or ground reaction changes.

Of the property parameters includable in the footwear properties, the grip indicates the amount of friction between the footwear 100 worn by a user in motion and the ground surface on which the footwear 100 landed or the amount of friction between the inside of the footwear 100 and a user's foot. The grip may be evaluated based on a performance evaluation result in terms of design and may also be evaluated in five grades of friction between the footwear 100 worn by a user in motion and the ground surface on which the footwear 100 landed. The grip may be evaluated by measuring, using a measuring device, friction between the footwear 100 worn by a user in motion and the ground surface on which the footwear 100 landed.

Of the property parameters includable in the footwear properties, the bendability indicates how easily the footwear 100 worn by a user is bendable when the user is in motion. The bendability may be evaluated based on a performance evaluation result in terms of design and may also be evaluated in five grades of how easily the footwear 100 worn by a user is bendable based on the sensation of a user in motion wearing the footwear 100. The bendability may be evaluated by measuring, using a measuring device, the extent of bending of the footwear 100 when a load is imposed on the footwear 100.

Of the property parameters includable in the footwear properties, the breathability indicates the amount of air passing through the footwear 100. The breathability may be evaluated based on a performance evaluation result in terms of design and may also be evaluated in five grades of how easily air is allowed to pass through the footwear 100 based on the sensation of a user wearing the footwear 100. The breathability may be evaluated by measuring, using a measuring device, the temperature and humidity of the footwear 100 and the amount of air passing through the footwear 100.

Of the property parameters includable in the footwear properties, the lightness in weight indicates the weight of the footwear 100. The lightness in weight may be evaluated based on a performance evaluation result in terms of design and may also be evaluated in five grades of the weight of the footwear 100 based on the sensation of a user wearing the footwear 100. The lightness in weight may be evaluated by measuring, using a measuring device, the weight of the footwear 100.

Of the property parameters includable in the footwear properties, the repulsion indicates the magnitude of a force that pushes back the footwear 100 when hitting the ground. The repulsion may be evaluated based on a performance evaluation result in terms of design, for example, load-displacement response during compression. The repulsion may also be evaluated in five grades of the dimension of a force that pushes back the footwear 100 when hitting the ground based on the sensation of a user wearing the footwear 100. Optionally, a weight may be dropped from a certain height onto the sole of the footwear 100 to measure a bounce-back height ratio (largest bounce-back height/initial height), which may also be used to evaluate the repulsion.

Of the property parameters includable in the footwear properties, the support against rapid moves and turns indicates the degree of stability when a user wearing the footwear 100 makes a turn to right or left. The support against rapid moves and turns may be evaluated based on a performance evaluation result in terms of design and may also be evaluated in five grades of the sensation of stability felt by a user wearing the footwear 100 when the user makes a turn to right or left. The support against rapid moves and turns may be evaluated by measuring, using image analysis, the ground reaction or the degree of lateral deformation of the footwear 100 when a user wearing the footwear 100 turns to right or left.

Of the property parameters includable in the footwear properties, the arch support indicates the sensation of support felt by a user for his/her foot arch when wearing the footwear 100. The arch support may be evaluated based on a performance evaluation result in terms of design and may also be evaluated in five grades of the sensation of support felt by a user for his/her foot arch when wearing the footwear 100. The arch support may be evaluated by measuring, using a measuring device, the size and/or hardness of an arch support portion.

The indicators used to evaluate the footwear properties and how to evaluate such indicators described thus far are just examples and are not necessarily limited to such. The property parameters of the footwear properties described herein are just examples and may be replaced with other property parameters that indicate typical footwear properties.

When the footwear properties (first property parameter) is inputted to the insole designing apparatus 1, in addition to simple input of the footwear property values, information, such as purpose, athletic game or sport in which the footwear is used, and/or a user's gender, age, weight, pronation, average running/walking speed, average running/walking distance, road surface condition, past injury(ies), may be further inputted, so that footwear properties suitably recommended for a user's habit of use are automatically inputted. In the insole designing apparatus 1, footwear properties obtained from a user's history of footwear orders may be stored in advance in the storage 13, so that the input of a footwear property value(s) to be desirably improved alone can be accepted based on the properties of the previously ordered footwear.

In the insole designing apparatus 1, footwear properties obtained from famous athletes and players may be stored in advance in the storage 13. Thus, the properties of footwear used by such famous athletes and players can be presented to assist the user when the user inputs the desired footwear properties. The insole designing apparatus 1 may allow the input of footwear properties by reading the properties of footwear worn by other athletes and players that a user accessed and obtained on the Internet. In the insole designing apparatus 1, footwear properties obtained from individuals with variously different habits of use may be stored in advance in the storage 13. Thus, any footwear properties similar to a user's habit of use can be read to assist the user when the user inputs the desired footwear properties. The insole designing apparatus 1 may be configured to allow a user to consult information such as footwear properties posted by purchasers and viewers on the social networking services (SNS) and/or online shops, thereby assisting the user when the user inputs the desired footwear properties.

The insole designing apparatus 1 receives footwear data of the footwear 100 in which the insole 1A will be used (second property parameter) (S203). The footwear data may preferably include at least one property parameter selected from the following; cushioning performance, stability, grip, bendability, fit, durability, breathability, lightness in weight, repulsion, support against rapid moves and turns, and arch support, so that the property parameter(s) can be compared to and analyzed against the footwear properties received in S202. The property parameters includable in the footwear data disclosed herein are just exemplified ones and may include other parameters. In the insole designing apparatus 1, the footwear data of the footwear 100 has been supplied from the manufacturer of the footwear 100 and is already stored in the storage 13. A user may directly input, to the insole designing apparatus 1, footwear data of the footwear 100 not stored yet in the storage 13, or the user may visit a shop(s) and evaluate the footwear 100 sold there and then input the footwear data to the insole designing apparatus 1.

The insole designing apparatus 1 calculates a differential value(s) between the footwear properties desired by the user and received in S202 and the footwear data received in S203 (S204). The insole designing apparatus 1 determines whether the differential value calculated in S204 of any parameter(s) of the footwear properties is greater than or equal to a predetermined value (S205). When the earlier step determines that the differential value of any parameter(s) of the footwear properties is greater than or equal to a predetermined value (YES in S205), the insole designing apparatus 1 adjusts the parameter(s) for adjustment included in the design data of the insole 1A for change to a parameter(s) of the footwear parameters as desired by user (S206).

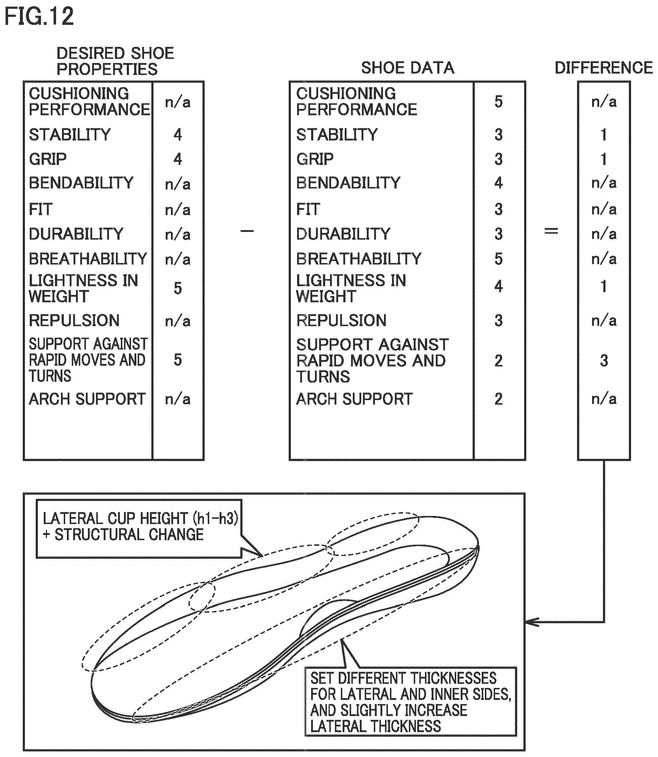

Examples are hereinafter described, in which the insole designing apparatus 1 adjusts the parameter(s) for adjustment included in the design data of the insole 1A so that the footwear data of the footwear 100 can be as close to the footwear properties desired by a user as possible. Fig. 12 is a schematic diagram for describing Example 1, illustrating adjustments of the design data using the insole designing apparatus 1 according to the embodiment. In Example 1, the insole 1A that can change the footwear properties is produced by the insole designing apparatus 1 when the footwear 100 purchased by a user are running shoes, and the user wants to wear the footwear 100 in an athletic game or sport that often demands rapid moves and turns in direction, like tennis or basketball.

First, the insole designing apparatus 1 receives the input of footwear properties required of an athletic game or sport like tennis or basketball desired by a user (S202). As illustrated in Fig. 12, the required footwear properties are; "4" for stability and grip, and "5" for lightness in weight and support against rapid moves and turns. Five grades are defined for the evaluation of footwear properties, ranging from the lowest "1" to the highest "5". Depending on the purpose of use, any irrelevant parameters of the footwear properties are rated as "n/a" and excluded from the criteria of evaluation. Of the footwear properties required of an athletic game or sport like tennis or basketball, the cushioning performance and durability are excluded from the criteria of evaluation in the example of Fig. 12.

The model number of the footwear 100 purchased by the user is inputted to the insole designing apparatus 1 to read, from the storage 13, the footwear data of the footwear 100 corresponding to the inputted model number. Then, the read footwear data is received by the insole designing apparatus 1 (S203). As illustrated in Fig. 12, the received footwear data of the footwear 100 is; "3" for stability and grip, "4" for lightness in weight, and "2" for support against rapid moves and turns. In the footwear data of the footwear 100, values for evaluation have been inputted to all of the parameters, as illustrated in Fig. 12, regardless of the current use of this footwear.

The insole designing apparatus 1 calculates the differential values between the required footwear properties and the footwear data of the footwear 100 (S204). As illustrated in Fig. 12, the calculated differential values are "1" for stability, grip and lightness in weight, and "3" for support against rapid moves and turns. The insole designing apparatus 1 determines that any parameter of the footwear properties with a differential value greater than or equal to "2" (predetermined value) needs to be changed (S205). As illustrated in Fig. 12, "3" is the differential value of the support against rapid moves and turns, which is greater than or equal to the predetermined value. Thus, the support against rapid moves and turns is determined as a parameter of the footwear properties that needs to be changed. Thus, any parameter of the footwear properties with a differential value greater than or equal to the predetermined value may be determined as a parameter that needs to be changed. Instead, any parameter of the footwear properties may be determined as a parameter that needs to be changed when its differential value is a minus value and the absolute value of this differential value is greater than or equal to "2" (predetermined value).

The insole designing apparatus 1 adjusts the design data of the insole 1A so that the parameters of the footwear properties can be as close to those of the footwear properties desired by the user as possible (S206). Among the design data of insole 1A, the structure, cup height in each portion and thickness in each portion are adjusted, so that the support against rapid moves and turns of the footwear 100, currently "2", can be as close to "5" as possible, as illustrated in Fig. 12. To ensure a better support against rapid moves and turns, the insole designing apparatus 1 adjusts the design data of the insole 1A, so that the insole is relatively hardened, the lateral cup heights (h1 to h3) are increased, and the lateral thicknesses are slightly increased to prevent the footwear 100 from leaning sideways.

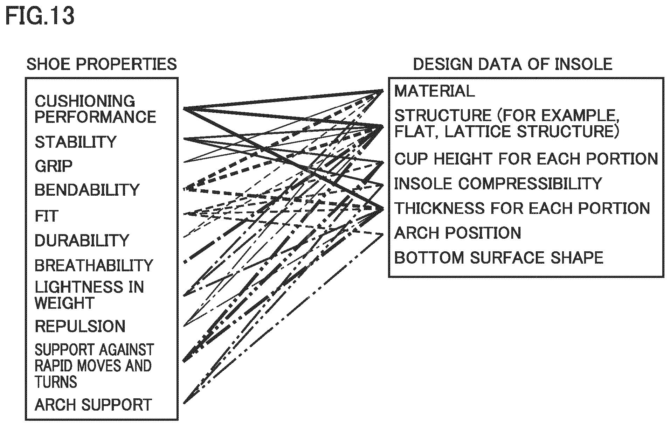

In the storage 13 of the insole designing apparatus 1 is previously stored data correlation, which helps to know which piece of design data of the insole 1A needs to be adjusted to change a particular one of the footwear property parameters. Fig. 13 is a diagram for describing correlation between the insole design data and footwear properties. The correlation illustrated in Fig. 13 is just an example. For example, at least one of the material, structure, and thickness in each portion in the design data of the insole 1A needs to be adjusted in order to change the cushioning performance among the parameters of footwear properties. In order to change the stability among the parameters of footwear properties, at least one of the following parameters may need to be adjusted; structure, thickness in each portion and insole scale ratio among the design data of the insole 1A. The insole designing apparatus 1 may flexibly set the amount of change of any parameter to be adjusted in view of a user's gender, age, weight, average walking/running speed, average running/walking distance, road surface condition, and/or past injury(ies).

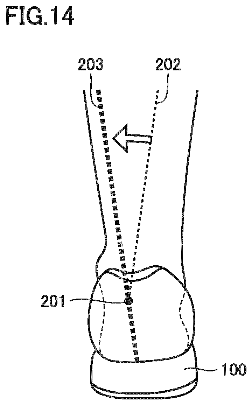

Example 2 describes designing, using the insole designing apparatus 1, the insole 1A that can change the footwear properties in case a user's foot pronation differs from the pronation of the footwear 100. Fig. 14 is a schematic diagram for describing pronation. The pronation refers to a natural motion of human body in which the heel of a foot leans inward at the time of hitting the ground. Specifically describing the pronation, a line 202 extending from a heel 201 before landing on the ground leans inward, like a line 203, under an impact when the foot hits the ground, as illustrated in Fig. 14. There are three groups of pronation classification; overpronation in which the line 203 makes a relatively large angle to the line 202, neutral pronation in which the line 203 makes a modest angle to the line 202, and underpronation in which the line 203 makes a relatively small angle to the line 202. If a user with overpronation feet wears the footwear 100 designed for neutral pronation, an excessive burden may be imposed on the user's legs and/or knees, or inadequate shock absorption may be likely to invite risks of pain and/or injury.

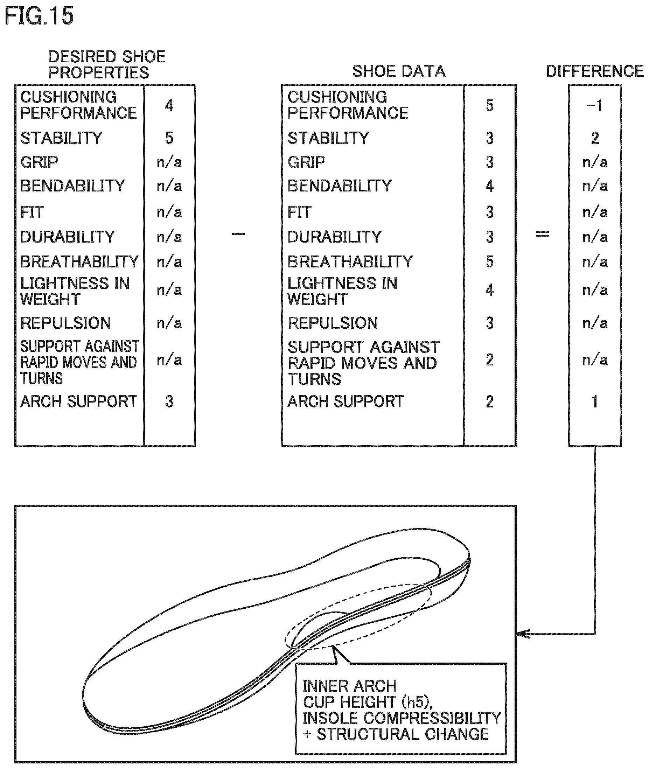

Fig. 15 is a schematic diagram for describing Example 2, illustrating adjustments of the design data using the insole designing apparatus 1 according to the embodiment. Fig. 15 describes an example in which the insole 1A is designed by the insole designing apparatus 1. In this example, the insole 1A, when used in the footwear 100, is allowed to adjust properties of the footwear 100 designed for neutral pronation, so that the footwear 100 is suitably worn by a user who walks/runs with overpronation feet.

First, overpronation footwear properties are received by the insole designing apparatus 1 as footwear properties desired by a user (S202). As illustrated in Fig. 15, required footwear properties are; "4" for cushioning performance, "5" for stability, and "3" for arch support. Five grades are defined for the evaluation of footwear properties, ranging from the lowest "1" to the highest "5". Depending on the purpose of use, any irrelevant parameters of the footwear properties are rated as "n/a" and excluded from the criteria of evaluation.

The model number of the footwear 100 purchased by the user is inputted to the insole designing apparatus 1 to read, from the storage 13, the footwear data of the footwear 100 corresponding to the inputted model number. Then, the read footwear data is received by the insole designing apparatus 1 (S203). As illustrated in Fig. 15, the received footwear data of the footwear 100 is; "5" for cushioning performance, "3" for stability, and "2" for arch support. In the footwear data of the footwear 100, values for evaluation have been inputted to all of the parameters, as illustrated in Fig. 15, regardless of the current use of this footwear.

The insole designing apparatus 1 calculates the differential values between the required footwear properties and the footwear data of the footwear 100 (S204). As illustrated in Fig. 15, the calculated differential values are; "-1" for cushioning performance, "2" for stability, and "1" for arch support. The insole designing apparatus 1 determines that any parameter of the footwear properties with a differential value greater than or equal to "2" (predetermined value) needs to be changed (S205). As illustrated in Fig. 15, "2" is the differential value in regard to the stability, which is within a range of values greater than or equal to the predetermined value. Thus, the stability is determined as a parameter of the footwear properties that needs to be changed.

The insole designing apparatus 1 adjusts the design data of the insole 1A to change the parameters of these footwear properties (S206). Among the design data of insole 1A, the structure, cup height in each portion and insole scale ratio are adjusted, so that the stability of the footwear 100, currently "3", can be as close to "5" as possible, as illustrated in Fig. 15. To prevent the heel from leaning inward when the foot hits the ground, the insole designing apparatus 1 adjusts the design data of the insole 1A so that the medial structure is relatively hardened, the cup height of the medial arch (h5) is increased, and the insole scale ratio on the medial side is slightly increased. The insole designing apparatus 1 may flexibly set the amount of change of any parameter to be adjusted in view of a user's gender, age, weight, average walking/running speed, average running/walking distance, road surface condition, and/or past injury(ies).

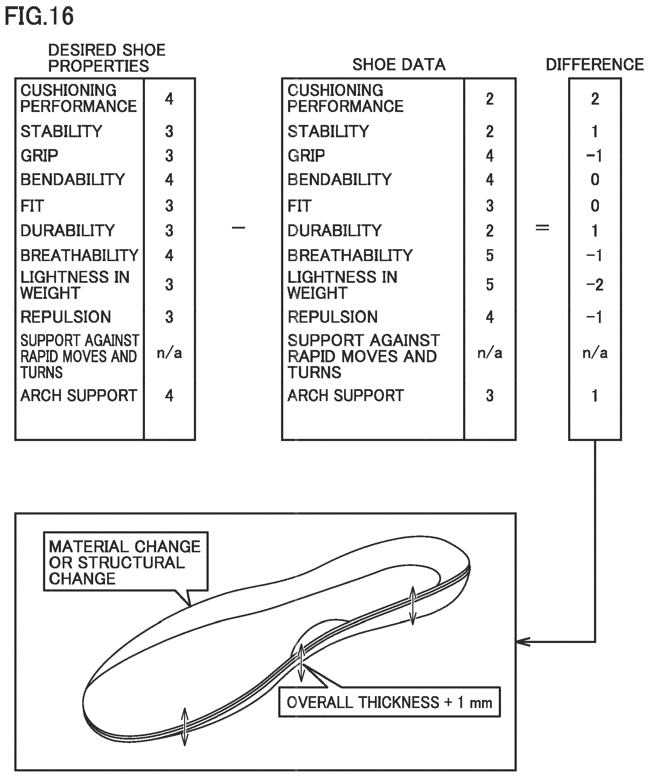

Next, Example 3 describes an example in which the insole 1A that can change the footwear properties is designed by the insole designing apparatus 1 in case the footwear 100 purchased by a user for day-to-day running practices are athletic shoes that are overengineered for the user. Fig. 16 is a schematic diagram for describing Example 3, illustrating adjustments of the design data using the insole designing apparatus 1 according to the embodiment. First, footwear properties for running practices are received by the insole designing apparatus 1 as footwear properties desired by a user (S202). As illustrated in Fig. 16, the required footwear properties are; "4" for cushioning performance, "3" for stability, "3" for grip, "4" for bendability, "3" for fit, "3" for durability, "4" for breathability, "3" for lightness in weight, "3" for repulsion, and "4" for arch support. Five grades are defined for the evaluation of footwear properties, ranging from the lowest "1" to the highest "5". Depending on the purpose of use, any irrelevant parameters of the footwear properties are rated as "n/a" and excluded from the criteria of evaluation.

The model number of the footwear 100 purchased by the user is inputted to the insole designing apparatus 1 to read, from the storage 13, the footwear data of the footwear 100 corresponding to the inputted model number. Then, the read footwear data is received by the insole designing apparatus 1 (S203). As illustrated in Fig. 16, the received footwear data of the footwear 100 are; "2" for cushioning performance, "2" for stability, "4" for grip, "4" for bendability, "3" for fit, "2" for durability, "5" for breathability, "5" for lightness in weight, "4" for repulsion, and "3" for arch support. Among the footwear data of the footwear 100 designed for running practices, the support against rapid moves and turns is rated as "n/a" and excluded from the criteria of evaluation.

The insole designing apparatus 1 calculates the differential values between the required footwear properties and the footwear data of the footwear 100 (S204). As illustrated in Fig. 16, the calculated differential values are; "2" for cushioning performance, "1" for stability, "-1" for grip, "0" for bendability, "0" for fit, "1" for durability, "-1" for breathability, "-2" for lightness in weight, "-1" for repulsion, and "1" for arch support. The insole designing apparatus 1 determines that any parameter of the footwear properties with a differential value greater than or equal to "2" (predetermined value) needs to be changed (S205). As illustrated in Fig. 16, "2" is the differential value in regard to the cushioning performance, which is within a range of values greater than or equal to the predetermined value. Thus, the cushioning performance is determined as a parameter of the footwear properties that needs to be changed.

The insole designing apparatus 1 adjusts the design data of the insole 1A to change the parameters of these footwear properties (S206). Among the design data of the insole 1A, the material, structure, and thickness in each portion are adjusted, so that the cushioning performance of the footwear 100, currently "2", can be as close to "4" as possible, as illustrated in Fig. 16. For better cushioning performance, the insole designing apparatus 1 may adjust the design data of the insole 1A, for example, select a more elastic material, employ a lattice structure, and/or increase the whole thickness by 1 mm. The lightness in weight has a minus differential value and the absolute value of this differential value is greater than or equal to "2" (predetermined value). This may be determined as a parameter of the footwear properties that needs to be changed. In this instance, the material, structure, and thickness in each portion, among the design data of the insole 1A, may be further adjusted. The insole designing apparatus 1 may flexibly set the amount of change of any parameter to be adjusted in view of a user's gender, age, weight, average walking/running speed, average running/walking distance, road surface condition, and/or past injury(ies).

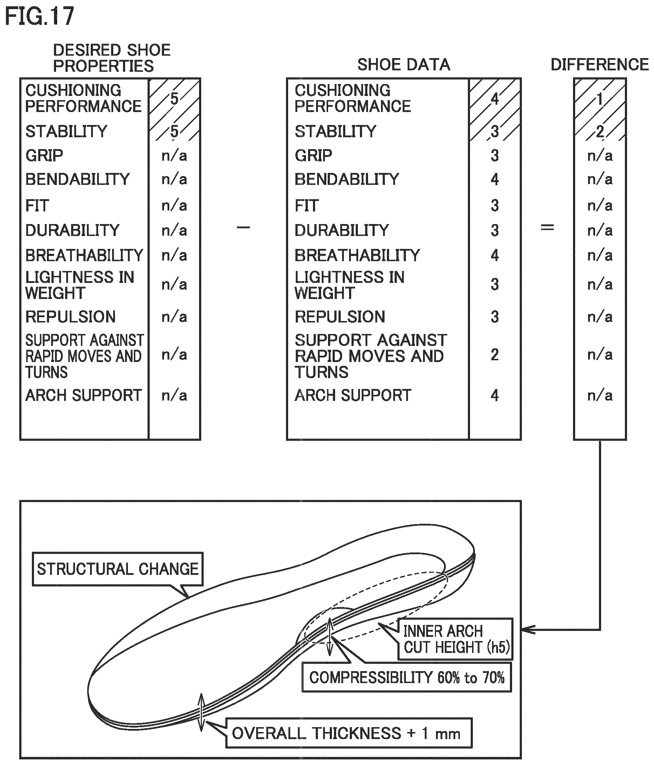

Example 4 describes designing, using the insole designing apparatus 1, the insole 1A that can change the footwear properties of the footwear 100 in case a user wants to further enhance the footwear properties selected earlier by the user. Fig. 17 is a schematic diagram for describing Example 4, illustrating adjustments of the design data using the insole designing apparatus 1 according to the embodiment. First, the footwear properties selected by a user are received by the insole designing apparatus 1 as footwear properties desired by the user (S202). As illustrated in Fig. 17, the required footwear properties are; "5" for cushioning performance, and "5" for stability. Five grades are defined for the evaluation of footwear properties, ranging from the lowest "1" to the highest "5". Any parameters not selected by the user are rated as irrelevant parameter "n/a" and excluded from the criteria of evaluation.

The model number of the footwear 100 purchased by the user is inputted to the insole designing apparatus 1 to read, from the storage 13, the footwear data of the footwear 100 corresponding to the inputted model number. Then, the read footwear data is received by the insole designing apparatus 1 (S203). As illustrated in Fig. 17, the received footwear data of the footwear 100 are; "4" for cushioning performance, "3" for stability, "3" for grip, "4" for bendability, "3" for fit, "3" for durability, "4" for breathability, "3" for lightness in weight, "3" for repulsion, "4" for arch support, and "2" for support against rapid moves and turns.

The insole designing apparatus 1 calculates the differential values between the required footwear properties and the footwear data of the footwear 100 (S204). As illustrated in Fig. 17, the calculated differential values are; "1" for cushioning performance, and "2" for stability. In Fig. 17, the parameters of footwear properties selected by the user are illustrated with hatched lines. The insole designing apparatus 1 determines that any parameter of the footwear properties with a differential value greater than or equal to "1" (predetermined value) needs to be changed (S205). As illustrated in Fig. 17, the differential values are; "1" for cushioning performance and "2" for stability, which are greater than or equal to a predetermined value. Thus, these values are determined as parameters of footwear properties that need to be changed.

The insole designing apparatus 1 adjusts the design data of the insole 1A to change the parameters of these footwear properties (S206). Among the design data of insole 1A, the material, structure, and thickness in each portion are adjusted, so that the cushioning performance of the footwear 100, currently "4", can be as close to "5" as possible, as illustrated in Fig. 17. Among the design data of insole 1A, the structure, thickness in each portion and insole scale ratio are further adjusted, so that the stability of the footwear 100, currently "3", can be as close to "5" as possible. To improve the cushioning performance, the insole designing apparatus 1 may adjust the design data of the insole 1A, for example, select a more elastic material, employ a lattice structure, and/or increase the whole thickness by 1 mm. For better stability, insole designing apparatus 1 may further adjust the design data of the insole 1A, for example, increase the cup height of the medial arch (h5) and/or change the insole scale ratio from 60% to 70%. The insole designing apparatus 1 may flexibly set the amount of change of any parameter to be adjusted in view of a user's gender, age, weight, average walking/running speed, average running/walking distance, road surface condition, and/or past injury(ies).

With reference to Fig. 11 again, in case all of the parameters of the footwear properties have been adjusted in S206 so that the differential values are less than the predetermined value, the insole designing apparatus 1 determines that none of the parameters of the footwear properties is greater than or equal to the predetermined value (NO in S205) and then outputs the adjusted design data of the insole 1A to the 3D printer 3 (or NC working machine) (S207). The 3D printer 3 (or NC working machine) produces the insole 1A based on the adjusted design data of the insole 1A. Then, the footwear 100 mounted with this insole 1A may be allowed to have footwear properties as close to the user's desired properties as possible.

### [Prediction of Foot Shape]

In S107 illustrated in Fig. 4, the insole designing apparatus 1 generates the design data of the insole 1A based on the foot form data received in S101. The foot form data received in S101 was measured by laser measuring unit 22 with a load being imposed on a subject's (user's) foot. To obtain the insole 1A well-adapted to the foot shape, however, foot form data is desirably obtained in an unloaded state in which the foot shape is unaffected by any load or in a low-load state with a less load than in the loaded state. Measuring the unloaded foot shape may involve an additional work requiring the use of, for example, a plaster bandage and is thus not a simple procedure. The insole designing apparatus 1 may perform a prediction process to predict a subject's unloaded foot shape from the subject's foot form data in the loaded state.

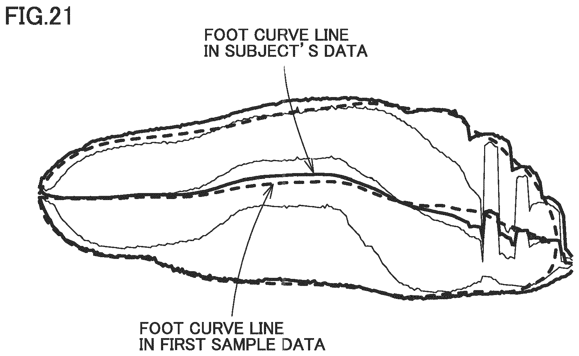

With reference to Figs. 18 to 21, the prediction process carried out by the insole designing apparatus 1 is hereinafter described, in which a subject's foot shape in the unloaded state is predicted. Fig. 18 is a flow chart of the prediction process to predict a subject's unloaded foot shape. Fig. 19 is a table showing an example of loaded foot shape data. Fig. 20 is a table showing an example of unloaded foot shape data. Fig. 21 is a diagram for describing the calculation of a difference between a subject's data and first sample data. Steps illustrated in Fig. 18 (hereinafter, step numbers starting with "S") are carried out by having the prediction program 131 run by the processor 11 of the insole designing apparatus 1.

As illustrated in Fig. 18, the insole designing apparatus 1 obtains the subject's data containing the subject's foot shape data in the loaded state measured by the measuring apparatus 2 (foot form data) (S301). The insole designing apparatus 1 produces a homology model based on the subject's data and extracts feature amounts of the subject's data (in this example, arch height ratio, heel leaning angle) (S302). The insole designing apparatus 1 selects the subject's foot shape type based on the feature amounts of the subject's data (S303). The insole designing apparatus 1 selects one of foot shape types illustrated in Fig. 19 classified based on subjects' foot arch types and heel leaning types.

The insole designing apparatus 1 extracts, from the loaded foot shape data 133 stored in the storage 13, first sample data in the loaded state that can adapt to the subject's foot shape type selected in S303 (S304). The first sample data thus extracted contains an average foot shape data in the loaded state that can adapt to the subject's foot shape type (homology model).

As illustrated in Fig. 19, the loaded foot shape data includes pieces of data obtained by classifying, for each of foot shape types, measured foot shape data of a plurality of samples in the loaded state (to be specific, homology model produced based on the measured data) based on at least one foot shape-related feature amount. In the example of Fig. 19, the arch height ratio and heel leaning angle (angle through which the heel leans inward) are used as at least one feature amount. Then, the loaded foot shape data is divided into nine different foot shape types according to three different arch types classified as per arch height ratio and three heel leaning types classified as per heel leaning angle.

The insole designing apparatus 1 extracts, from the unloaded foot shape data 134 stored in the storage 13, second sample data in the unloaded state that can adapt to the subject's foot shape type selected in S303 (S305). The second sample data thus extracted includes an average foot shape data (homology model) in the unloaded state that can adapt to the subject's foot shape type.

As illustrated in Fig. 20, the unloaded foot shape data includes pieces of data obtained by classifying, for each of different foot shape types, measured foot shape data of a plurality of samples in the unloaded state (to be specific, homology model produced based on the measured data) based on at least one foot shape-related feature amount. In the example of Fig. 20, the arch height ratio and heel leaning angle are used as at least one feature amount. Then, the unloaded foot shape data is divided into nine different foot shape types according to three different arch types classified as per arch height ratio and three heel leaning types classified as per heel leaning angle.

The insole designing apparatus 1 adjusts the foot length and orthogonal foot width of the first sample data in the loaded state extracted in S304 to be equal to the foot length and orthogonal foot width of the homology model produced based on the subject's data (S306). Thus, the curve line of an average foot in the loaded state corresponding to the first sample data is changed in accordance with the subject's foot length and orthogonal foot width.

The insole designing apparatus 1 adjusts the foot length and orthogonal foot width of the second sample data in the unloaded state extracted in S305 to be equal to the foot length and orthogonal foot width of the homology model produced based on the subject's data (S307). Thus, the curve line of an average foot in the unloaded state corresponding to the second sample data is changed in accordance with the subject's foot length and orthogonal foot width.

The insole designing apparatus 1 calculates the foot curve line based on the subject's data and the first sample data changed in S306 and compares the foot curve line in the subject's data with the foot curve line in the loaded state corresponding to the first sample data changed in S306 to calculate a difference between these foot curve lines (S308).

More specifically, the insole designing apparatus 1 compares the foot curve line in the subject's data illustrated with a solid line and the foot curve line in the loaded state corresponding to the first sample data illustrated with a dotted line to calculate a difference between these two curve lines in the direction of foot length, as illustrated in Fig. 21.

The foot curve line of the subject's data and the foot curve line of the first sample data in the loaded state only need to be calculated from the foot's sole shape obtained by the measuring apparatus 2. More specifically, a contour line, medial ground contact line and lateral ground contact line may be determinable even in the loaded state. Therefore, the foot curve line of the subject's data and the foot curve line of the first sample data in the loaded state only need to be calculated based on the contour line, medial ground contact line and lateral ground contact line. The foot curve line of the second sample data in the unloaded state may be used instead of the foot curve line of the first sample data. For instance, the medial ground contact line and lateral ground contact line may be calculated using a point of intersection of a line passing through at a certain height from a lowest line with an outer shape on the medial or lateral side of foot, the foot curve line of the second sample data may be calculated using the calculated medial ground contact line and lateral ground contact line, so that the curve line thus calculated is used as the foot curve line of the first sample data.

The insole designing apparatus 1 changes the degree of foot bending (curve line) in the unloaded state corresponding to the second sample data adjusted in S306 based on the calculated curve line difference to calculate the subject's foot shape (homology model) in the unloaded state (S309). Then, the insole designing apparatus 1 ends this process.

Specifically, in the insole designing apparatus 1, a plurality of component points along and constituting the foot's outer shape in cross section in the second sample data are shifted in a direction in which the difference calculated in S308 can be cancelled. More specifically, in the insole designing apparatus 1, a plurality of component points along and constituting the foot's outer shape in cross section in the second sample data are shifted so that the foot curve line in the second sample data is coincident with the foot curve line in the subject's data. The insole designing apparatus 1 thus shifts these component points constituting the foot's outer shape at predetermined intervals (for example, at the intervals of 1 mm) along the direction of foot length.

Thus, the insole designing apparatus 1 is allowed to predict the subject's foot shape data in the unloaded state (homology model) from data of the subject's foot shape in the loaded state measured by the measuring apparatus 2. Thus, the subject's foot shape data in the unloaded state is predicted from data of the subject's foot shape in the loaded state, which, however, is just an exemplified method of prediction. For example, the insole designing apparatus 1 may generate a prediction model through training by machine learning using training data and then predict the subject's foot shape in the unloaded state from the measured data using the training-based prediction model.

### [Modified example]

The present disclosure may encompass, in its scope, variously different modifications. Modified examples applicable to the technology disclosed herein are described below.

As illustrated in Fig. 11, the insole designing apparatus 1 according to the embodiment determines whether any parameter of the footwear properties needs to be changed based on whether any difference between the received footwear properties (first property parameter) and footwear data (second property parameter) is greater than or equal to a predetermined value.

The insole designing apparatus 1 may otherwise determine whether any parameter of the footwear properties needs to be changed. For example, the apparatus may multiply, by a coefficient, any difference between the received footwear properties (first property parameter) and footwear data (second property parameter) and then compare the resulting value with a predetermined value, or may execute a computing process by assigning the difference to a predetermined function.

The insole designing apparatus 1 may be installed in a store or shop equipped with the measuring apparatus 2 or may be a cloud-based server device. The insole designing apparatus 1, if it is a cloud-based server device, may be connected to the measuring apparatuses 2 installed in stores and shops and generate the design data of the insole 1A based on pieces of foot shape data obtained from these measuring apparatuses 2.

## Claims

1. An insole designing apparatus (1) for use in designing an insole (1A) of footwear (100), the insole designing apparatus (1) comprising:
an input device (14) that is configured to receive an input of data;
a processing circuitry (11) that is configured to calculate design data of the insole (1A) based on the data received by the input device (14); and
an output device (14) that is configured to output the design data calculated by the processing circuitry (11),
wherein the data received by the input device (14) at least includes first data associated with foot shape of a user and second data associated with an inside of the footwear (100),
the processing circuitry (11) is configured to calculate the design data based on the first data and the second data, and
the second data includes inside data of the footwear (100),
**characterized in that**
the processing circuitry (11) is configured to obtain the design data of a contour (M1) of the insole (1A) by modelling by
selecting a base model of the insole (1A) most approximate to an inner curving shape of the footwear (100) based on the obtained inside data of the footwear (100),
enlarging or reducing the base model based on the first data, and
deforming the base model, so that the interval from each of a plurality of points of control of a standard contour (M) of the base model to the inside of the footwear (100) is reduced to a shortest distance.

2. The insole designing apparatus (1) according to claim 1, wherein the second data includes data obtained by scanning or capturing an image of the inside of the footwear (100) mounted with the insole (1A) and data of a scanned shape or an image-captured shape of the insole (1A) of the footwear (100).

3. The insole designing apparatus (1) according to claim 1, further comprising a storage (13) in which the second data is storable in association with product information of the footwear (100),
wherein the processing circuitry (11) is configured to read the second data from the storage (13) based on the product information of the footwear (100) received by the input device (14).

4. The insole designing apparatus (1) according to claim 3, wherein in case where the size of the footwear (100) received by the input device (14) differs from the size of the footwear (100) in the second data stored in the storage (13), the processing circuitry (11) is configured to adjust the size of the footwear (100) in the second data.

5. The insole designing apparatus (1) according to claim 1, wherein
the input device (14) is further configured to receive:
a first property parameter indicating properties of the footwear (100) desired by the user; and
a second property parameter indicating properties of the footwear (100) in which the insole (1A) will be used by the user,
the processing circuitry (11) is configured to adjust the design data based on a difference between the first property parameter and the second property parameter received by the input device (14).

6. The insole designing apparatus (1) according to claim 5, wherein the first property parameter and the second property parameter include at least one of: cushioning performance; stability; grip; bendability; fit; durability; breathability; lightness in weight; repulsion; support against rapid moves and turns; and arch support.

7. The insole designing apparatus (1) according to claim 5, wherein the design data includes at least one of: material; structure; cup height in each portion; scale ratio of the insole (1A); thickness in each portion; arch position; and bottom surface shape.

8. The insole designing apparatus (1) according to claim 5, wherein the processing circuitry (11) is configured to adjust the design data, so that the second property parameter having a differential value to the first property parameter, the differential value being greater than or equal to a predetermined value, is adjusted to have a value close to the first property parameter.

9. The insole designing apparatus (1) according to claim 5, wherein the processing circuitry (11) is configured to adjust the design data, so that the second property parameter has a value greater than or equal to the first property parameter desired by the user.

10. The insole designing apparatus (1) according to claim 1, wherein the first data contains a foot shape data of the user in a loaded state,
the processing circuitry (11) is configured to predict a foot shape of the user in an unloaded state based on the foot shape data of the user in the loaded state to calculate the design data of the insole (1A) based on the foot shape predicted earlier.

11. The insole designing apparatus (1) according to any one of claims 1 to 10, wherein the design data is data associated with the insole (1A) including a three-dimensional mesh structure.

12. An insole designing method for use in designing an insole (1A) of footwear (100), the method comprising:
receiving at least first data associated with a foot shape of a user and second data associated with an inside of the footwear (100), including inside data of the footwear (100);
calculating design data of the insole (1A) based on the first data and the second data received earlier;
outputting the design data thus calculated,
**characterized by**;
obtaining the design data of a contour (M1) of the insole (1A) by modelling by
selecting a base model of the insole (1A) most approximate to an inner curving shape of the footwear (100) based on the obtained inside data of the footwear (100),
enlarging or reducing the base model based on the first data, and
deforming the base model, so that the interval from each of a plurality of points of control of a standard contour (M) of the base model to the inside of the footwear (100) is reduced to a shortest distance.

13. A program for use in an insole designing apparatus (1) that designs an insole (1A) of footwear (100), the insole designing apparatus (1) including:
an input device (14) that is configured to receive an input of data;
a processing circuitry (11) that is configured to calculate design data of the insole (1A) based on the data received by the input device (14), the program being executable by the processing circuitry (11); and
an output device (14) that is configured to output the design data calculated by the processing circuitry (11),
the program comprising:
receiving, by the input device (14), at least first data associated with foot shape of a user and second data associated with an inside of the footwear (100);
calculating the design data of the insole (1A) based on the first data and the second data received by the input device (14);
outputting the design data calculated earlier from the output device (14),
**characterized by**
obtaining the design data of a contour (M1) of the insole (1A) by modelling by
selecting a base model of the insole (1A) most approximate to an inner curving shape of the footwear (100) based on the obtained inside data of the footwear (100),
enlarging or reducing the base model based on the first data, and
deforming the base model, so that the interval from each of a plurality of points of control of a standard contour (M) of the base model to the inside of the footwear (100) is reduced to a shortest distance.

## Patentansprüche

1. Einlegesohlengestaltungssvorrichtung (1) zur Verwendung beim Gestalten einer Einlegesohle (1A) von Schuhwerk (100), wobei die Einlegesohlengestaltungsvorrichtung (1) umfasst:
ein Eingabegerät (14), das konfiguriert ist, um eine Eingabe von Daten zu empfangen;
eine Verarbeitungsschaltung (11), die konfiguriert ist, um Gestaltungsdaten der Einlegesohle (1A) auf Grundlage der von dem Eingabegerät (14) empfangenen Daten zu berechnen; und
ein Ausgabegerät (14), das konfiguriert ist, um die von der Verarbeitungsschaltung (11) berechneten Gestaltungsdaten auszugeben,
wobei die von dem Eingabegerät (14) empfangenen Daten mindestens erste Daten, die mit der Fußform eines Benutzers assoziiert sind, und zweite Daten, die mit einem Inneren des Schuhwerks (100) assoziiert sind, aufweisen.
die Verarbeitungsschaltung (11) konfiguriert ist, um die Gestaltungsdaten auf Grundlage der ersten Daten und der zweiten Daten berechnet, und
die zweiten Daten Innendaten des Schuhwerks (100) aufweisen,
**dadurch gekennzeichnet, dass**
die Verarbeitungsschaltung (11) konfiguriert ist, um die Gestaltungsdaten einer Kontur (M1) der Einlegesohle (1A) durch Modellierung zu erhalten, durch
Auswählen eines Basismodells der Einlegesohle (1A), das einer inneren Krümmungsform des Schuhwerks (100) am nächsten kommt, auf Grundlage der erhaltenen Innendaten des Schuhwerks (100),
Vergrößern oder Verkleinern des Basismodells auf Grundlage der ersten Daten, und
Verformen des Basismodells, so dass das Intervall von jedem einer Vielzahl von Kontrollpunkten einer Standardkontur (M) des Basismodells zu dem Inneren des Schuhwerks (100) auf eine kürzeste Distanz reduziert wird.

2. Einlegesohlengestaltungssvorrichtung (1) nach Anspruch 1, wobei die zweiten Daten Daten aufweisen, die durch Scannen oder Erfassen eines Bildes der Innenseite des mit der Einlegesohle (1A) versehenen Schuhwerks (100) erhalten werden und Daten einer gescannten Form oder einer Bild erfassten Form der Einlegesohle (1A) des Schuhwerks (100).

3. Einlegesohlengestaltungssvorrichtung (1) nach Anspruch 1, die ferner einen Speicher (13) umfasst, in dem die zweiten Daten in Verbindung mit Produktinformationen des Schuhwerks (100) speicherbar sind,
wobei die Verarbeitungsschaltung (11) konfiguriert ist, um die zweiten Daten aus dem Speicher (13) auf Grundlage der von dem Eingabegerät (14) empfangenen Produktinformationen des Schuhwerks (100) zu lesen.

4. Einlegesohlengestaltungssvorrichtung (1) nach Anspruch 3, wobei in einem Fall, in dem die von dem Eingabegerät (14) empfangene Größe des Schuhwerks (100) von der Größe des Schuhwerks (100) in den im Speicher (13) gespeicherten zweiten Daten abweicht, die Verarbeitungsschaltung (11) konfiguriert ist, um die Größe des Schuhwerks (100) in den zweiten Daten anzupassen.

5. Einlegesohlengestaltungssvorrichtung (1) nach Anspruch 1, wobei
das Eingabegerät (14) ferner konfiguriert ist, um zu empfangen:
einen ersten Eigenschaftsparameter, der von dem Benutzer gewünschte Eigenschaften des Schuhwerks (100) angibt; und
einen zweiten Eigenschaftsparameter, der Eigenschaften des Schuhwerks (100) angibt, in dem die Einlegesohle (1A) von dem Benutzer verwendet werden wird,
die Verarbeitungsschaltung (11) konfiguriert ist, um die Gestaltungsdaten auf Grundlage einer Differenz zwischen dem ersten Eigenschaftsparameter und dem zweiten Eigenschaftsparameter, die von der Eingabevorrichtung (14) empfangen werden, anzupassen.

6. Einlegesohlengestaltungssvorrichtung (1) nach Anspruch 5, wobei der erste Eigenschaftsparameter und der zweite Eigenschaftsparameter mindestens eines aufweist von: Dämpfungsleistung; Stabilität; Griffigkeit; Biegsamkeit; Passform; Langlebigkeit; Atmungsaktivität; geringes Gewicht; Rückstoß; Unterstützung bei schnellen Bewegungen und Drehungen; und Fußgewölbeuntertützung.

7. Einlegesohlengestaltungssvorrichtung (1) nach Anspruch 5, wobei die Gestaltungsdaten mindestens eines aufweisen von: Material; Struktur; Schalenhöhe in jedem Abschnitt; Skalierungsverhältnis der Einlegesohle (1A); Dicke in jedem Abschnitt; Fußgewölbeposition; und Bodenflächenform.

8. Einlegesohlengestaltungssvorrichtung (1) nach Anspruch 5, wobei die Verarbeitungsschaltung (11) konfiguriert ist, um die Gestaltungsdaten anzupassen, so dass der zweite Eigenschaftsparameter, der einen Differenzwert zu dem ersten Eigenschaftsparameter hat, wobei der Differenzwert größer oder gleich einem vorbestimmten Wert ist, angepasst wird, um einen Wert nahe dem ersten Eigenschaftsparameter zu haben.

9. Einlegesohlengestaltungssvorrichtung (1) nach Anspruch 5, wobei die Verarbeitungsschaltung (11) konfiguriert ist, um die Gestaltungsdaten anzupassen, so dass der zweite Eigenschaftsparameter einen Wert hat, der größer oder gleich dem vom Benutzer gewünschten ersten Eigenschaftsparameter ist.

10. Einlegesohlengestaltungssvorrichtung (1) nach Anspruch 1, wobei die ersten Daten Fußformdaten des Benutzers in einem belasteten Zustand enthalten,
die Verarbeitungsschaltung (11) konfiguriert ist, um eine Fußform des Benutzers in einem unbelasteten Zustand auf Grundlage der Fußformdaten des Benutzers in dem belasteten Zustand vorherzusagen, um die Gestaltungsdaten der Einlegesohle (1A) auf Grundlage der zuvor vorhergesagten Fußform zu berechnen.

11. Einlegesohlengestaltungssvorrichtung (1) nach einem der Ansprüche 1 bis 10, wobei die Gestaltungsdaten Daten sind, die mit der Einlegesohle (1A) assoziiert sind, die eine dreidimensionale Netzstruktur aufweist.

12. Einlegesohlengestaltungsverfahren zur Verwendung beim Gestalten einer Einlegesohle (1A) von Schuhwerk (100), wobei das Verfahren umfasst:
Empfangen von mindestens ersten Daten, die mit einer Fußform eines Benutzers assoziiert sind, und zweiten Daten, die mit einem Inneren des Schuhwerks (100) assoziiert sind, einschließlich Innendaten des Schuhwerks (100);
Berechnen von Gestaltungsdaten der Einlegesohle (1A) auf Grundlage der zuvor empfangenen ersten und zweiten Daten;
Ausgeben der so berechneten Gestaltungsdaten,
**gekennzeichnet durch**;
Erhalten der Gestaltungsdaten einer Kontur (M1) der Einlegesohle (1A) durch Modellierung durch
Auswählen eines Basismodells der Einlegesohle (1A), das einer inneren Krümmungsform des Schuhwerks (100) am nächsten kommt, auf Grundlage der erhaltenen Innendaten des Schuhwerks (100),
Vergrößern oder Verkleinern des Basismodells auf der Grundlage der ersten Daten, und
Verformen des Basismodells, so dass das Intervall von jedem einer Vielzahl von Kontrollpunkten einer Standardkontur (M) des Basismodells zu dem Inneren des Schuhwerks (100) auf eine kürzeste Distanz reduziert wird.

13. Programm zur Verwendung in einer Einlegesohlengestaltungssvorrichtung (1), die eine Einlegesohle (1A) von Schuhwerk (100) gestaltet, wobei die Einlegesohlengestaltungssvorrichtung (1) aufweist:
ein Eingabegerät (14), das konfiguriert ist, um eine Eingabe von Daten zu empfangen;
eine Verarbeitungsschaltung (11), die konfiguriert ist, um Gestaltungsdaten der Einlegesohle (1A) auf Grundlage der von dem Eingabegerät (14) empfangenen Daten zu berechnen, wobei das Programm durch die Verarbeitungsschaltung (11) ausführbar ist; und
ein Ausgabegerät (14), das konfiguriert ist, um die von der Verarbeitungsschaltung (11) berechneten Gestaltungsdaten auszugeben,
wobei das Programm umfasst:
Empfangen, durch die Eingabevorrichtung (14), von mindestens ersten Daten, die mit einer Fußform eines Benutzers assoziiert sind, und zweiten Daten, die mit einem Inneren des Schuhwerks (100) assoziiert sind;
Berechnen der Gestaltungsdaten der Einlegesohle (1A) auf Grundlage der ersten Daten und der zweiten Daten, die von der Eingabevorrichtung (14) empfangen werden;
Ausgabe der zuvor berechneten Gestaltungsdaten von dem Ausgabegerät (14),
**gekennzeichnet durch**
Erhalten der Gestaltungsdaten einer Kontur (M1) der Einlegesohle (1A) durch Modellieren durch
Auswählen eines Basismodells der Einlegesohle (1A), das einer inneren Krümmungsform des Schuhwerks (100) am nächsten kommt, auf Grundlage der erhaltenen Innendaten des Schuhwerks (100),
Vergrößern oder Verkleinern des Basismodells auf der Grundlage der ersten Daten, und
Verformen des Basismodells, so dass das Intervall von jedem einer Vielzahl von Kontrollpunkten einer Standardkontur (M) des Basismodells zu dem Inneren des Schuhwerks (100) auf eine kürzeste Distanz reduziert wird.

## Revendications

1. Appareil de conception de semelles intérieures (1) à utiliser pour concevoir une semelle intérieure (1A) de chaussures (100), l'appareil de conception de semelles intérieures (1) comprenant :
un dispositif d'entrée (14) configuré pour recevoir une entrée de données ;
un circuit de traitement (11) configuré pour calculer des données de conception de la semelle intérieure (1A) sur la base des données reçues par le dispositif d'entrée (14) ; et
un dispositif de sortie (14) configuré pour sortir les données de conception calculées par le circuit de traitement (11),
dans lequel les données reçues par le dispositif d'entrée (14) comprennent au moins des premières données associées à une forme de pied d'un utilisateur et des secondes données associées à un intérieur de la chaussure (100),
le circuit de traitement (11) est configuré pour calculer les données de conception sur la base des premières données et des secondes données, et
les secondes données comportent des données d'intérieur de la chaussure (100),
**caractérisé en ce que**
le circuit de traitement (11) est configuré pour obtenir les données de conception d'un contour (M1) de la semelle intérieure (1A) par modélisation en
sélectionnant un modèle de base de la semelle intérieure (1A) le plus proche d'une forme de courbure intérieure de la chaussure (100) sur la base des données d'intérieur obtenues de la chaussure (100),
élargissant ou réduisant le modèle de base sur la base des premières données, et
déformant le modèle de base, de sorte que l'intervalle depuis chacun d'une pluralité de points de contrôle d'un contour standard (M) du modèle de base à l'intérieur de la chaussure (100) soit réduit à la distance la plus courte.

2. Appareil de conception de semelles intérieures (1) selon la revendication 1, dans lequel les secondes données comportent des données obtenues par balayage ou capture d'une image de l'intérieur de la chaussure (100) montée avec la semelle intérieure (1A) et des données d'une forme issue d'un balayage ou d'une forme capturée par l'image de la semelle intérieure (1A) de la chaussure (100).

3. Appareil de conception de semelles intérieures (1) selon la revendication 1 comprenant en outre une mémoire (13) dans laquelle les secondes données peuvent être stockées en association avec des informations de produit de la chaussure (100),
dans lequel le circuit de traitement (11) est configuré pour lire les secondes données de la mémoire (13) sur la base des informations de produit de la chaussure (100) reçues par le dispositif d'entrée (14).

4. Appareil de conception de semelles intérieures (1) selon la revendication 3, dans lequel, dans un cas où la taille de la chaussure (100) reçue par le dispositif d'entrée (14) diffère de la taille de la chaussure (100) dans les secondes données stockées dans la mémoire (13), le circuit de traitement (11) est configuré pour ajuster la taille de la chaussure (100) dans les secondes données.

5. Appareil de conception de semelles intérieures (1) selon la revendication 1, dans lequel
le dispositif d'entrée (14) est en outre configuré pour recevoir :
un premier paramètre de propriété indiquant des propriétés de la chaussure (100) souhaitées par l'utilisateur ; et
un second paramètre de propriété indiquant des propriétés de la chaussure (100) dans laquelle la semelle intérieure (1A) sera utilisée par l'utilisateur,
le circuit de traitement (11) est configuré pour ajuster les données de conception sur la base d'une différence entre le premier paramètre de propriété et le second paramètre de propriété reçus par le dispositif d'entrée (14).

6. Appareil de conception de semelles intérieures (1) selon la revendication 5, dans lequel le premier paramètre de propriété et le second paramètre de propriété comportent au moins un parmi : performance d'amortissement ; stabilité ; adhérence ; flexibilité ; ajustement ; durabilité ; respirabilité ; légèreté en poids ; répulsion ; soutien contre les mouvements et les virages rapides ; et soutien de la voûte plantaire.

7. Appareil de conception de semelles intérieures (1) selon la revendication 5, dans lequel les données de conception comportent au moins un parmi : matériau, structure, hauteur de cuvette dans chaque partie, rapport d'échelle de la semelle intérieure (1A), épaisseur dans chaque partie, position de la voûte plantaire et forme de la surface inférieure.

8. Appareil de conception de semelles intérieures (1) selon la revendication 5, dans lequel le circuit de traitement (11) est configuré pour ajuster les données de conception, de sorte que le second paramètre de propriété ayant une valeur différentielle par rapport au premier paramètre de propriété, la valeur différentielle étant supérieure ou égale à une valeur prédéterminée, est ajusté pour avoir une valeur proche du premier paramètre de propriété.

9. Appareil de conception de semelles intérieures (1) selon la revendication 5, dans lequel le circuit de traitement (11) est configuré pour ajuster les données de conception, de sorte que le second paramètre de propriété ait une valeur supérieure ou égale au premier paramètre de propriété souhaité par l'utilisateur.

10. Appareil de conception de semelles intérieures (1) selon la revendication 1, dans lequel les premières données contiennent une donnée de forme de pied de l'utilisateur dans un état chargé,
le circuit de traitement (11) est configuré pour prédire une forme de pied de l'utilisateur dans un état déchargé sur la base des données de forme de pied de l'utilisateur dans l'état chargé, afin de calculer les données de conception de la semelle intérieure (1A) sur la base de la forme de pied prédite plus tôt.

11. Appareil de conception de semelles intérieures (1) selon l'une quelconque des revendications 1 à 10, dans lequel les données de conception sont des données associées à la semelle (1A) comportant une structure de maille tridimensionnelle.

12. Procédé de conception de semelles intérieures à utiliser pour concevoir une semelle intérieure (1A) d'une chaussure (100), le procédé comprenant :
réception d'au moins premières données associées à une forme de pied d'un utilisateur et de secondes données associées un intérieur de la chaussure (100), comportant des données d'intérieur de la chaussure (100) ;
calcul des données de conception de la semelle intérieure (1A) sur la base des premières données et des secondes données reçues plus tôt ;
sortie des données de conception ainsi calculées,
**caractérisé par**
obtention des données de conception d'un contour (M1) de la semelle intérieure (1A) par modélisation en
sélectionnant un modèle de base de la semelle intérieure (1A) le plus proche d'une forme de courbure intérieure de la chaussure (100) sur la base des données d'intérieur obtenues de la chaussure (100),
élargissant ou réduisant le modèle de base sur la base des premières données, et
déformant le modèle de base, de sorte que l'intervalle depuis chacun d'une pluralité de points de contrôle d'un contour standard (M) du modèle de base à l'intérieur de la chaussure (100) soit réduit à la distance la plus courte.

13. Programme pour utilisation dans un appareil de conception de semelles intérieures (1) qui conçoit une semelle intérieure (1A) de chaussures (100), l'appareil de conception de semelles intérieures (1) comportant :
un dispositif d'entrée (14) configuré pour recevoir une entrée de données ;
un circuit de traitement (11) configuré pour calculer des données de conception de la semelle intérieure (1A) sur la base des données reçues par le dispositif d'entrée (14), le programme étant exécutable par le circuit de traitement (11) ; et
un dispositif de sortie (14) configuré pour sortir les données de conception calculées par le circuit de traitement (11),
le programme comprenant :
réception, par le dispositif d'entrée (14), d'au moins des premières données associées à la forme de pied d'un utilisateur et les secondes données associées à un intérieur de la chaussure (100) ;
calcul des données de conception de la semelle intérieure (1A) sur la base des premières données et des secondes données reçues par le dispositif d'entrée (14) ;
sortie des données de conception calculées plus tôt à partir du dispositif de sortie (14),
**caractérisé par**
obtention des données de conception d'un contour (M1) de la semelle intérieure (1A) par modélisation en
sélectionnant un modèle de base de la semelle intérieure (1A) le plus proche d'une forme de courbure intérieure de la chaussure (100) sur la base des données d'intérieur obtenues de la chaussure (100),
élargissant ou réduisant le modèle de base sur la base des premières données, et
déformant le modèle de base, de sorte que l'intervalle depuis chacun d'une pluralité de points de contrôle d'un contour standard (M) du modèle de base à l'intérieur de la chaussure (100) soit réduit à la distance la plus courte.
